# EUROPEAN PATENT SPECIFICATION

(11) **EP 4 171 586 B1**
(45) Date of publication and mention of the grant of the patent: **16.04.2025**
(21) Application number: 21748708.1
(22) Date of filing: 28.06.2021
(51) Int. Cl.: A61K 35/17, C12N 5/0783

(54) **POLY-DONOR CD4+ T CELLS EXPRESSING IL-10 AND USES THEREOF**
POLYDONOR CD4+ T-ZELLEN, DIE IL-10 EXPRIMIEREN, UND VERWENDUNGEN DAVON
CELLULES T CD4+ POLYDONATRICES EXPRIMANT L'IL-10 ET LEUR UTILISATION

(30) Priority: 30.06.2020 WO PCT/US2020/040372
(43) Date of publication of application: 03.05.2023
(73) Proprietor: TR1X, Inc., San Diego, CA 92121 (US)
(72) Inventor: Roncarolo, Maria, Grazia, La Jolla, CA 92037 (US); de Vries, Jan, Egbert, La Jolla, CA 92037 (US)
(74) Representative: Titmus, Craig Edward
(86) International application number: PCT/US2021/039464
(87) International publication number: WO 2022/006020

(56) References cited:
- WO-A1-2013/192215
- WO-A1-2016/146542
- WO-A1-2019/002633
- US-A1- 2015 132 272
- GRAZIA ANDOLFI ET AL: "Enforced IL-10 Expression Confers Type 1 Regulatory T Cell (Tr1) Phenotype and Function to Human CD4+ T Cells", MOLECULAR THERAPY, vol. 20, no. 9, 1 September 2012 (2012-09-01), US, pages 1778 - 1790, XP055592834, ISSN: 1525-0016, DOI: 10.1038/mt.2012.71
- VAN MONTFRANS CATHERINE ET AL: "Generation of regulatory gut-homing human T lymphocytes using ex vivo interleukin 10 gene transfer", GASTROENTEROLOGY, vol. 123, no. 6, 2002 - 2002, pages 1877 - 1888, XP029482907, ISSN: 0016-5085, DOI: 10.1053/GAST.2002.37066
- B. M. SEGAL ET AL: "Cutting Edge: IL-10-Producing CD4+ T Cells Mediate Tumor Rejection", THE JOURNAL OF IMMUNOLOGY, vol. 168, no. 1, 1 January 2002 (2002-01-01), US, pages 1 - 4, XP055269949, ISSN: 0022-1767, DOI: 10.4049/jimmunol.168.1.1
- OH JAE-WON ET AL: "CD4 T-helper cells engineered to produce IL-10 prevent allergen-induced airway hyperreactivity and inflammation", JOURNAL OF ALLERGY AND CLINICAL IMMUNOLOGY, vol. 110, no. 3, 1 September 2002 (2002-09-01), AMSTERDAM, NL, pages 460 - 468, XP055852256, ISSN: 0091-6749, DOI: 10.1067/mai.2002.127512

## Description

### 1. CROSS REFERENCE TO RELATED APPLICATIONS

The instant application claims priority to PCT application, PCT/US2020/040372 filed on June 30, 2020.

### 2. SEQUENCE LISTING

The instant application contains a Sequence Listing with 5 sequences.

### 3. BACKGROUND

Regulatory T cells belong to a small but important subset of T cells which maintain immunological tolerance to self and non-pathogenic antigens and maintain immune homeostasis. There are two major populations of regulatory T cells - CD4⁺, Foxp3⁺ CD25⁺ T cells (Foxp3⁺ cells) and type 1 regulatory T (Tr1) cells. Both Foxp3⁺ and Tr1 cells downregulate pathogenic T-cell responses in various preclinical models for organ and pancreatic islet transplantation, GvHD and various autoimmune and inflammatory diseases.

Tr1 cells have shown to be effective in clinical studies. Administration of cloned, antigen-specific, autologous Tr1 cells to patients with ongoing moderate to severe Crohn's disease resulted in objective, transient remissions (Desreumaux et al., Gastroenterology. 2012;143(5):1207-1217.e2.). In addition, adoptive transfer of donor-derived allo-specific CD4⁺ T cell populations enriched for Tr1 cells to leukemia patients following allogeneic HSCT resulted in a rapid reconstitution of the immune system and protection against microbial and viral infections, without severe GvHD. In the responder patients, long term remissions and tolerance (> 7 years) resulting in cures were achieved (Bacchetta et al., Front Immunol. 2014;5:16).

Despite these encouraging results, the production of donor-derived or autologous Tr1 cells for large scale therapy for patients with high unmet medical needs is not always feasible, is very cumbersome, and also does not allow for the generation of large quantities of pure Tr1 cells.

Recently, Locafaro and colleagues circumvented some of these problems by transducing purified CD4⁺ T cells from a single donor with a bidirectional lentiviral vector containing a human IL-10 gene. The resulting single-donor CD4^{IL-10} populations shared the major functions of naturally occurring Tr1 cells. Like Tr1 cells, single-donor CD4^{IL-10} cells produce high levels of IL-10 and downregulate the proliferation of both allogeneic CD4⁺ and CD8⁺ T cells. In addition, they are cytotoxic for both normal myeloid cells (including antigen presenting cells, APC) and myeloid leukemia cells. These single-donor CD4^{IL-10} cells were shown to be effective in reducing GvHD in a humanized xeno-GvHD model while retaining graft-versus-leukemia (GvL) activity. *See* Locafaro et al. Mol Ther. 2017;25(10):2254-2269 and WO 2016/146,542.

Although it is possible to produce highly purified single-donor CD4^{IL-10} cells for therapeutic use, there are still significant limitations, because of qualitative and quantitative differences between the various individual batches, which most likely are related to intrinsic differences between the various donors in addition to variations in the quality of buffy coats.

### 1. SUMMARY

The invention is defined according to the claims. Thus, the invention provides a population of CD4⁺ T cells that have been genetically modified to comprise an exogenous polynucleotide encoding IL-10, wherein: (a) the CD4⁺ T cells were obtained from at least three different T cell donors (poly-donor CD4^{IL-10} cells); and (b) the exogenous polynucleotide is integrated into the T cell nuclear genome.

In some embodiments, the CD4⁺ T cells in the population collectively have six, seven, eight, nine, ten, eleven, twelve, or more different HLA haplotypes. In some embodiments: (a) all the CD4⁺ T cells in the population have: (i) at least 5/10, 6/10, 7/10, 8/10, or 9/10 match at the HLA-A, HLA-B, HLA-C, HLA-DRB1, and HLA-DQB1 loci to each other; (ii) at least 4/8, 5/8, 6/8, 7/8, or 8/8 match at the HLA-A, HLA-B, HLA-C, and HLA-DRB 1 loci to each other; (iii) 2/2 match at the HLA-A locus to each other; (iv) 2/2 match at the HLA-B locus to each other; (v) 2/2 match at the HLA-C locus to each other; and/or (vi) all the CD4⁺ T cells in the population have at least 3/4 or 4/4 match at the HLA-DRB1 and HLA-DQB1 loci with each other; and/or (b) all the CD4⁺ T cells in the population have an A*02 allele.

In some embodiments, the exogenous polynucleotide further comprises: (a) a sequence encoding a selection marker, optionally wherein the selection marker is ΔNGFR; and/or (b) lentiviral vector sequences.

In some embodiments, (a) at least 90% of the CD4⁺ T cells within the population express IL-10; (b) the genetically modified CD4⁺ T cells constitutively express at least 100pg, 200pg, 500pg, 1ng, 5ng, 10ng, or 50ng IL-10 per 10⁶ of the CD4⁺ T cells/ml of culture medium; and/or (c) the genetically modified CD4⁺ T cells express at least 1ng, 2ng, 5ng, 10ng, 100ng, 200ng, or 500ng IL-10 per 10⁶ of the CD4⁺ T cells/ml after activation with anti-CD3 and anti-CD28 antibodies. In some embodiments, the exogenous polynucleotide further comprises a sequence encoding a selection marker and at least 90%, at least 95%, or at least 98% of the CD4⁺ T cells within the population express the selection marker from the exogenous polynucleotide.

In some embodiments, the genetically modified CD4⁺ T cells express CD49b, LAG-3, TGF-β, IFNy, GzB, perforin, CD18, CD2, CD226, and/or IL-22.

In some embodiments, the CD4⁺ T cells are in: (a) a frozen suspension; or (b) a liquid suspension, optionally wherein the liquid suspension has previously been frozen.

The invention also provides a method of making poly-donor CD4^{IL-10} cells, comprising the steps of: (i) modifying primary CD4⁺ T cells which have been obtained from at least three different T cell donors, wherein each donor's CD4⁺ T cells are separately modified by introducing an exogenous polynucleotide encoding IL-10, and (ii) pooling the genetically modified CD4⁺ T cells, thereby obtaining the poly-donor CD4^{IL-10} cells.

In some embodiments, the method further comprises the step, before step (i), after step (i), after step (i) and before step (ii), or after step (ii), of: incubating the primary CD4⁺ T cells in the presence of: (a) an anti-CD3 antibody, and anti-CD28 antibody; or (b) anti-CD3 antibody and anti-CD28 antibody coated beads, optionally wherein the method further comprises incubating the primary CD4⁺ T cells in the presence of IL-2.

In some embodiments, the exogenous polynucleotide further comprises a segment encoding a selection marker, optionally wherein the encoded selection marker is ΔNGFR; optionally wherein the method further comprises the step, after step (i), of: isolating the genetically-modified CD4⁺ T cells expressing the selection marker, thereby generating an enriched population of genetically-modified CD4⁺ T cells.

In some embodiments, in step (i), the primary CD4⁺ T cells are obtained from one or more frozen stocks.

The invention also provides the population of CD4⁺ cells according to the invention for use in a method of treating a patient in need of immune tolerization, wherein the method comprises the step of: administering the poly-donor CD4^{IL-10} cells to the patient.

In some embodiments, the method further comprises the step of: administering hematopoietic stem cells (HSC) of an HSC donor to the patient either prior to or subsequent to administration of the pooled donor CD4^{IL-10} cells.

In some embodiments, the patient has: (a) an inflammatory or autoimmune disease, optionally wherein the inflammatory or autoimmune disease is selected from the group consisting of type-1 diabetes, Crohn's disease, autoimmune uveitis, rheumatoid arthritis, psoriasis, psoriatic arthritis, multiple sclerosis, systemic lupus, inflammatory bowel disease, Addison's disease, Graves' disease, Sjögren's syndrome, Hashimoto's thyroiditis, myasthenia gravis, autoimmune vasculitis, pernicious anemia, ulcerative colitis, bullous diseases, scleroderma, and celiac disease; (b) a disease or disorder involving hyperactivity of NLPR3 inflammasome; (c) type 2 diabetes, a neurodegenerative disease, a cardiovascular disease or inflammatory bowel disease; and/or (d) an allergic or atopic disease, optionally wherein the allergic or atopic disease is selected from the group consisting of: asthma, atopic dermatitis, and rhinitis.

In some embodiments, the method further comprises the step of organ transplantation to the patient, either prior to or subsequent to administration of the population of CD4⁺ T cells.

The invention also provides the population of CD4⁺ cells according to the invention for use in a method of treating a hematological cancer, the method comprising: administering to a hematological cancer patient an amount of poly-donor CD4^{IL-10} cells sufficient to induce anti-cancer effect, wherein the poly-donor CD4^{IL-10} cells are genetically modified by vector-mediated gene transfer of the coding sequence of human IL-10 under control of a constitutive promoter.

In some embodiments, (a) the poly-donor CD4^{IL-10} cells are administered to the patient prior to or subsequent to administration of an allogenic hematopoietic stem cell transplant (allo HSCT), and the amount of poly-donor CD4^{IL-10} cells administered to the patient is sufficient to suppress GvHD without suppressing graft versus leukemia (GvL) or graft versus tumor (GvT) efficacy of the allo HSCT; and/or (b) the hematological cancer is a myeloid leukemia.

In some embodiments, the patient has Crohn's disease.

The present disclosure provides a new Tr1-based therapy using a population of poly-donor CD4^{IL-10} cells. Poly-donor CD4^{IL-10} cells refer to CD4⁺ T cells obtained from at least three different T cell donors and then genetically modified to comprise an exogenous polynucleotide encoding IL-10. The T cell donors are third party donors who are neither a host to be treated with the poly-donor CD4^{IL-10} cells nor an HSC or organ transplant donor. The poly-donor CD4^{IL-10} cells are not alloantigen-specific, i.e., they have not been primed or stimulated with cells from the host before administration.

Applicant demonstrated that the poly-donor CD4^{IL-10} cells have cytokine production profiles, immune suppressive- and cytotoxic capabilities comparable to those of single-donor CD4^{IL-10} cells. In addition, *in vivo,* they are more effective in preventing xeno GvHD mediated by CD4+ T cells than single-donor CD4^{IL-10} cells, while they do not induce GvHD by themselves. Overall, the functional properties of these poly-donor CD4^{IL-10} cells both *in vitro* and *in vivo* were comparable to or better than those of single donor CD4^{IL-10} cells.

Based on these results, Applicant claims that poly-donor allogeneic CD4^{IL-10} cells can be used for therapeutic purposes in GvHD, cell and organ transplantation, autoimmune- and inflammatory diseases.

Further, by using third party T cells and eliminating the requirement of allo-specificity, poly-donor CD4^{IL-10} cells makes the Tr1-based cell therapy available to a larger population of patients with various genetic backgrounds.

Accordingly, the present invention provides a population of CD4⁺T cells that have been genetically modified to comprise an exogenous polynucleotide encoding IL-10, wherein the CD4⁺ T cells were obtained from at least three different T cell donors (poly-donor CD4^{IL-10} cells) and the exogenous polynucleotide is integrated into the T cell nuclear genome.

The CD4⁺ T cells may be obtained from three, four, five, six, seven, eight, nine, or ten different T cell donors. In some embodiments, the CD4⁺ T cells in the population collectively have six, seven, eight, nine, ten, eleven, twelve, or more different HLA haplotypes.

In some embodiments, all the CD4⁺ T cells in the population have at least 5/10, 6/10, 7/10, 8/10, or 9/10 match at the HLA-A, HLA-B, HLA-C, HLA-DRB1, and HLA-DQB1 loci to each other. In some embodiments, all the CD4⁺ T cells in the population have at least 4/8, 5/8, 6/8, 7/8, or 8/8 match at the HLA-A, HLA-B, HLA-C, and HLA-DRB1 loci to each other. In some embodiments, all the CD4⁺ T cells in the population have 2/2 match at the HLA-A locus to each other. In some embodiments, all the CD4⁺ T cells in the population have 2/2 match at the HLA-B locus to each other. In some embodiments, all the CD4⁺ T cells in the population have 2/2 match at the HLA-C locus to each other. In some embodiments, all the CD4⁺ T cells in the population have at least 3/4 or 4/4 match at the HLA-DRB1 and HLA-DQB1 loci with each other. In some embodiments, all the CD4⁺ T cells in the population have an A*02 allele.

In some instances, none of the CD4⁺ T cells is immortalized. The exogenous polynucleotide may comprise an IL-10-encoding polynucleotide segment operably linked to expression control elements. The IL-10 may be a human IL-10. The IL-10 may be a viral IL-10. The IL-10-encoding polynucleotide segment may encode a protein having the sequence of SEQ ID NO:1. The IL-10-encoding polynucleotide segment may have the sequence of SEQ ID NO:2. The expression control elements may drive constitutive expression of the encoded IL-10. The expression control elements may drive expression of IL-10 in activated CD4⁺ T cells. The expression control elements may drive tissue- specific or CD4⁺ T cell-specific expression.

In some embodiments, the exogenous polynucleotide further comprises a sequence encoding a selection marker. In some embodiments, the selection marker is ΔNGFR. The ΔNGFR may have the sequence of SEQ ID NO: 3. The exogenous polynucleotide may comprise a sequence of SEQ ID NO:4. The exogenous polynucleotide may have a sequence of SEQ ID NO: 5.

The selection marker may be a truncated EGFR polypeptide. The selection marker may be a truncated human EGFR polypeptide.

In the CD4⁺ T cell population of the invention, the exogenous polynucleotide is integrated into the T cell nuclear genome. In some embodiments, the exogenous polynucleotide further comprises lentiviral vector sequences.

In some embodiments, at least 90% of the CD4⁺ T cells within the population express IL-10. In some embodiments, at least 95% of the CD4⁺ T cells within the population express IL-10. In some embodiments, at least 98% of the CD4⁺ T cells within the population express IL-10.

In some embodiments, the genetically modified CD4⁺ T cells constitutively express at least 100pg IL-10 per 10⁶ of the CD4⁻ T cells/ml of culture medium. In some embodiments, the genetically modified CD4⁺ T cells constitutively express at least 200pg, 500pg, 1ng, 5ng, 10ng, or 50ng IL-10 per 10⁶ of the CD4⁺ T cells/ml. In some embodiments, the genetically modified CD4⁺ T cells express at least 1or 2ng IL-10 per 10⁶ of the CD4⁺ T cells/ml after activation with anti-CD3 and anti-CD28 antibodies. In some embodiments, the genetically modified CD4⁺ T cells express at least 2ng, 5ng, 10ng, 100ng, 200ng, or 500ng IL-10 per 10⁶ of the CD4⁺ T cells/ml after activation with anti-CD3 and anti-CD28 antibodies. The genetically modified CD4⁺ T cells may express IL-10 at a level at least 5-fold higher than unmodified CD4⁺ T cells. The genetically modified CD4⁺ T cells may express IL-10 at a level at least 10-fold higher than unmodified CD4⁺ T cells.

In some embodiments, at least 90% of the CD4⁺ T cells within the population express the selection marker from the exogenous polynucleotide. In some embodiments, at least 95% of the CD4⁺ T cells within the population express the selection marker from the exogenous polynucleotide. In some embodiments, at least 98% of the CD4' T cells within the population express the selection marker from the exogenous polynucleotide.

In some embodiments, the genetically modified CD4⁺ T cells express CD49b. In some embodiments, the genetically modified CD4⁺ T cells express LAG-3. In some embodiments, the genetically modified CD4⁺ T cells express TGF-β. In some embodiments, the genetically modified CD4⁺ T cells express IFNy. In some embodiments, the genetically modified CD4⁺ T cells express GzB. In some embodiments, the genetically modified CD4⁻ T cells express perforin. In some embodiments, the genetically modified CD4⁺ T cells express CD18. In some embodiments, the genetically modified CD4⁺ T cells express CD2. In some embodiments, the genetically modified CD4⁺ T cells express CD226. In some embodiments, the genetically modified CD4⁺ T cells express IL-22.

In some instances, the CD4⁺ T cells have not been anergized in the presence of peripheral blood mononuclear cells (PBMCs) from a host. In some instances, the CD4⁺ Tcells have not been anergized in the presence of recombinant IL-10 protein, wherein the recombinant IL-10 protein is not expressed from the CD4' T cells. In some instances, the CD4' T cells have not been anergized in the presence of DC10 cells from a host.

In some embodiments, the CD4⁺ T cells are in a frozen suspension. In some embodiments, the CD4⁺ T cells are in a liquid suspension. In some embodiments, the liquid suspension has previously been frozen.

Also described herein is a pharmaceutical composition comprising:
(i) the population of CD4⁺ T cells as defined in the claims; suspended in
(ii) a pharmaceutically acceptable carrier.

Also described herein is a method of making poly-donorCD4^{IL-10} cells, comprising the steps of:
(i) pooling primary CD4⁺ T cells obtained from at least three different T cell donors; and
(ii) modifying the pooled CD4⁺ T cells by introducing an exogenous polynucleotide encoding IL-10,
thereby obtaining the genetically-modified CD4⁺ T cells.

In one aspect, the present invention provides a method of making poly-donor CD4^{IL-10} cells, comprising the steps of:
(i) modifying primary CD4+ T cells which have been obtained from at least three different T cell donors, wherein each donor's CD4⁺ T cells are separately modified by introducing an exogenouspolynucleotide encoding IL-10, and
(ii) pooling the genetically modified CD4⁺ T cells,
thereby obtaining the genetically-modified CD4⁺ T cells.

In some embodiments, the method further comprises the step, before step (i), after step (i), after step (i) and before step (ii), or after step (ii), of:
incubating the primary CD4⁺ T cells in the presence of an anti-CD3 antibody, and anti-CD28 antibody or anti-CD3 antibody and CD28 antibody coated beads.

In some embodiments, the method comprises incubating the primary CD4⁺ T cells further in the presence of IL-2. The exogenous polynucleotide may be introduced into the primary CD4⁺ T cells using a viral vector. The viral vector may be a lentiviral vector. The exogenous polynucleotide may comprise a segment encoding IL-10 having the sequence of SEQ ID NO:1. The IL-10- encoding polynucleotide segment may have the sequence of SEQ ID NO:2.

In some embodiments, the exogenous polynucleotide further comprises a segment encoding a selection marker. In some embodiments, the encoded selection marker is ΔNGFR. The encoded selection marker may have the sequence of SEQ ID NO:3. The encoded selection marker may be a truncated EGFR polypeptide. The encoded selection marker may be a truncated human EGFR polypeptide.

In some embodiments, the method further comprises the step, after step (i), of:
isolating the genetically-modified CD4⁺ T cells expressing the selection marker, thereby generating an enriched population of genetically-modified CD4⁺ T cells.

In some embodiments, at least 90% or at least 95% of the genetically-modified CD4⁺ T cells in the enriched population express IL-10. In some embodiments, at least 98% of the genetically-modified CD4⁺ T cells in the enriched population express IL-10. In some embodiments, at least 90% or at least 95% of the genetically-modified CD4⁺ T cells in the enriched population express the selection marker. In some embodiments, at least 98% of the genetically-modified CD4⁺ T cells in the enriched population express the selection marker.

The method may further comprise the step of incubating the enriched population of genetically-modified CD4' T cells. The step of incubating the enriched population of genetically-modified CD4⁺ T cells may be performed in the presence of anti-CD3 antibody and anti-CD28 antibody or CD3 antibody and CD28 antibody coated beads inthe presence of IL-2.

The method may further comprise the later step of freezing the genetically-modified CD4⁺ T cells. The primary CD4⁺ T cells may have been obtained from three, four, five, six, seven, eight, nine, or ten different T cell donors. In some embodiments, the at least three T cell donors have at least 5/10, 6/10, 7/10, 8/10, or 9/10 match at the HLA-A, HLA-B, HLA-C, HLA-DRB1, and HLA-DQB1 loci to each other. In some embodiments, the at least three T cell donors have at least 4/8, 5/8, 6/8, 7/8, or 8/8 match at theHLA-A, HLA-B, HLA-C, and HLA-DRB1 loci to each other. In some embodiments, the at least three T cell donors have 2/2 match at the HLA-A locus to each other. In some embodiments, the at least three T cell donors have 2/2 match at the HLA-B locus to each other. In some embodiments, the at least three T cell donors have 2/2 match at the HLA-C locus to each other. In some embodiments, the at least three T cell donors have at least 3/4 or 4/4 match at the HLA- DRB1 and HLA-DQB1 loci to each other. In some embodiments, each of the at least three T cell donors has an A*02 allele.

In some embodiments, in step (i), the primary CD4⁺T cells are obtained from one or more frozen stocks. In step (i), the primary CD4⁺T cells may be obtained from unfrozen peripheral blood mononuclear cells of the at least three different T cell donors.

The method may further comprise the step of isolating CD4⁺ T cells from the peripheral blood mononuclear cells. The peripheral blood mononuclear cells may be obtained from buffy coat or apheresis.

In another aspect, the present invention provides the population of CD4⁺
cells as defined in the claims for use in a method of treating a patient in need of immune tolerization, the method comprising the step of:
administering the poly-donor CD4^{IL-10} cells or a pharmaceutical composition of thepresent disclosure to the patient.

The method may further comprise the preceding step of thawing afrozen suspension of poly-donor CD4^{IL-10} cells.

The poly-donor CD4^{IL-10} cells or the pharmaceutical composition may prevent or reduce severity of pathogenic T cell response in the patient.

The method may further comprise the step of administering mononuclear cells from a hematopoictic stem cells (HSC) donor to the patient. The poly-donor CD4^{IL-10} cells or the pharmaceutical composition and the mononuclear cells from a HSC donor may be administered concurrently. The mononuclear cells from a HSC may be administered either prior to or subsequent to administration of the poly-donor CD4^{IL-10} cells or the pharmaceutical composition. The mononuclear cells may be in the PBMC. The mononuclear cells may be in the bone marrow. The mononuclear cells may be in the cord blood. The mononuclear cells may have been isolated from the PBMC, bone marrow or cord blood.

In some embodiments, the method further comprises the step of:
administering hematopoietic stem cells (HSC) of an HSC donor to the patient either prior to or subsequent to administration of the poly-donor CD4^{IL-10} cells or pharmaceutical composition.

The HSC donor may be partially HLA-mismatched to the patient. The HSC donor may have less than 5/10, 6/10, 7/10, 8/10, 9/10 or 10/10 match at the HLA-A, HLA-B, HLA-C, HLA-DRB1, and HLA-DQB1 loci to the patient. The HSC donor may have less than 4/8, 5/8,6/8, 7/8, or 8/8 match at the HLA-A, HLA-B,HLA-C, and HLA-DRB1 loci to the patient. The HSC donor may have less than2/2 match at the HLA-A, HLA-B, or HLA-C locus to the patient. The HSC donor may have less than 3/4 or 4/4 match at the HLA-DRB1 and HLA-DQB1 loci to the patient.

One or more of the T cell donors may be HLA-mismatched or partially HLA-mismatched to the patient. One or more of the T cell donors may have less than 5/10, 6/10, 7/10, 8/10, 9/10 or 10/10 match at the HLA-A, HLA-B, HLA-C, HLA- DRB1, and HLA-DQB1 loci to the patient. One or more of the T cell donors may have less than 4/8, 5/8, 6/8, 7/8, or 8/8 match at the HLA-A, HLA-B, HLA-C, and HLA- DRB1 loci to the patient. One or more of the T cell donors may have less than2/2 match at the HLA-A, HLA-B, or HLA-C locus to the patient. One or more of the T cell donors may have less than 2/4, 3/4 or 4/4 match at the HLA-DRB1 and HLA- DQB1 loci to the patient. One or more of the T cell donors may be HLA- mismatched or partially HLA-mismatched with the HSC donor. One or more of the T cell donors may have less than 5/10, 6/10, 7/10, 8/10, 9/10 or 10/10 match at the HLA- A, HLA-B, HLA-C, HLA-DRB1, and HLA-DQB1 loci to the HSC donor. One or more of the T cell donors may have less than 4/8, 5/8, 6/8, 7/8, or 8/8 match at the HLA-A, HLA-B, HLA-C, and HLA-DRB1 loci to the HSC donor. One or more of the T cell donors may have less than 2/2 match at the HLA-A, HLA-B, or HLA-C locus to the HSC donor. One or more of the T cell donors may have less than3/4 or 4/4 match at the HLA-DRB1 and HLA-DQB1 loci to the HSC donor.

In some embodiments, the poly-donor CD4^{IL-10} cells or the pharmaceutical composition prevents or reduces severity of GvHD by the transplanted hematopoietic stem cells.

The poly-donor CD4^{IL-10} cells or the pharmaceutical composition may prevent or reduce severity of pathogenic response of lymphoid cells from the transplanted hematopoietic stem cells.

The patient may have a cancer. The patient may have neoplastic cells. The neoplastic cells may express CD13, HLA-class I and CD54. The neoplastic cells may express CD112, CD58, or CD155.

The patient may have a cancer. The cancer may be a solid or hematological neoplasm. The patient may have a cancer selected from the group consisting of: Adrenal Cancer, Anal Cancer, Bile Duct Cancer, Bladder Cancer, Bone Cancer, Brain/CNS Tumors In Adults, Brain/CNS Tumors In Children, Breast Cancer, Breast Cancer In Men, Cancer of Unknown Primary, Castleman Disease, Cervical Cancer, Colon/Rectum Cancer, Endometrial Cancer, Esophagus Cancer, Ewing Family Of Tumors, Eye Cancer, Gallbladder Cancer, Gastrointestinal Carcinoid Tumors, Gastrointestinal Stromal Tumor(GIST), Gestational Trophoblastic Disease, Hodgkin Disease, Kaposi Sarcoma, Kidney Cancer, Laryngeal and Hypopharyngeal Cancer, Leukemia, Acute Lymphocytic (ALL), Acute Myeloid (AML, including myeloid sarcoma and leukemia cutis), Chronic Lymphocytic (CLL), ChronicMycloid (CML) Leukemia, Chronic Myelomonocytic (CMML), Leukemia in Children, Liver Cancer, Lung Cancer, Lung Cancer with Non-Small Cell, Lung Cancer with Small Cell, Lung Carcinoid Tumor, Lymphoma, Lymphoma of the Skin, Malignant Mesothelioma, Multiple Myeloma, Myelodysplastic Syndrome, Nasal Cavity and Paranasal Sinus Cancer, Nasopharyngeal Cancer, Neuroblastoma, Non-Hodgkin Lymphoma, Non-Hodgkin Lymphoma In Children, Oral Cavity and Oropharyngeal Cancer, Osteosarcoma, Ovarian Cancer, Pancreatic Cancer, Penile Cancer, Pituitary Tumors, Prostate Cancer, Retinoblastoma, Rhabdomyosarcoma, Salivary Gland Cancer, Sarcoma - Adult Soft Tissue Cancer, Skin Cancer, Skin Cancer - Basaland Squamous Cell, Skin Cancer - Melanoma, Skin Cancer - Merkel Cell, Small Intestine Cancer, Stomach Cancer, Testicular Cancer, Thymus Cancer, Thyroid Cancer, Uterine Sarcoma, Vaginal Cancer, Vulvar Cancer, Waldenstrom Macroglobulinemia, and Wilms Tumor.

The cancer may be a myeloid cancer. The cancer may be AML or CML.

In some embodiments, the patient has an inflammatory or autoimmune disease. In some embodiments, the inflammatory or autoimmune disease is selected from the group consisting of: type-1 diabetes, autoimmune uveitis, rheumatoid arthritis, psoriasis, psoriatic arthritis, multiple sclerosis, systemic lupus, inflammatory bowel disease, Addison's disease, Graves' disease, Sjögren's syndrome, Hashimoto's thyroiditis, myasthenia gravis, autoimmune vasculitis, pernicious anemia, ulcerative colitis, bullous diseases, scleroderma, and celiac disease.

The inflammatory or autoimmune disease may be Crohn's disease,ulcerative colitis, celiac disease, type-1 diabetes, lupus, psoriasis, psoriatic arthritis, or rheumatoid arthritis.

In some embodiments, the patient has a disease or disorder involving hyperactivity of NLPR3 inflammasome. In some embodiments, the patient has type 2 diabetes, a neurodegenerative disease, or inflammatory bowel disease.

The patient may have a disease or disorder involving increased IL-1β production by activated monocytes, macrophages or dendritic cells.

The patient may have a disease or disorder involving increased IL-18 production by activated monocytes, macrophages or dendritic cells.

The patient may have a disease or disorder involving increased mature caspase 1 production by activated monocytes, macrophages or dendritic cells.

In some embodiments, the patient has an allergic or atopic disease. In some embodiments, the allergic or atopic disease is selected from the group consisting of : asthma, atopic dermatitis, and rhinitis. The patient may have a food allergy.

In some embodiments, the method further comprises the step of cell and organ transplantation to the patient, either prior to or subsequent to administration of the population of CD4⁺ T cells or the pharmaceutical composition. In some embodiments, the poly-donor CD4^{IL-10} cells or the pharmaceutical composition prevents or reduces severity of host rejection of the cell and organ transplantation.

The method may further comprise the step of transplanting iPS cell-derived cells or tissues to the patient, either prior to or subsequent to administration of the population of CD4⁺ T cells or the pharmaceutical composition.

Poly-donor CD4^{IL-10} cells or the pharmaceutical composition may prevent or reduce severity of host rejection of the transplantation.

The method may further comprise the step of administering a recombinant AAV to the patient, either prior to or subsequent to administration of the poly-donorCD4^{IL-10} cells or the pharmaceutical composition. The poly-donor CD4^{IL-10} cells or the pharmaceutical composition may reduce immune responses against the recombinant AAV.

The patient may have an excessive immune response against viral orbacterial infection. The patient may have a coronavirus infection.

The method may further comprise the step of detecting the selection marker in a biological sample obtained from the patient, thereby detecting presence or absence ofpoly-donor CD4^{IL-10} T cells. The biological sample may be a biopsy or blood from the patient.

Also described herein is a population of CD4⁻ cells as defined in the claims for use in a method of treating a patient with a malignancy, the method comprising: administering an allo-HSCT graft to the patient, and administering atherapeutically effective amount of poly-donor CD4^{IL-10} cells.

In some instances, none of the donors of the CD4^{IL-10} cells in the poly-donor CD4^{IL-10} cells is the donor of the HSCT graft.

In another aspect, the invention provides the population of CD4⁺ cells as defined in the claims for use in a method of treating a hematological cancer, the method comprising: administering to a hematological cancer patient an amount of poly-donor CD4^{IL-10} cells sufficient to induce anti-cancer effects, wherein the poly-donor CD4^{IL-10} cells have been genetically modified by vector-mediated gene transfer of the coding sequence of human IL-10 under control of a constitutive promoter.

The method may further comprise the step of administering allo HSCT graft to the patient prior to or subsequence to administration of the poly-donor CD4^{IL-10} cells. In some embodiments, the amount of poly-donor CD4^{IL-10} cells administered to the patient is sufficient to suppress graft-versus-host disease (GvHD) without suppressing graft-versus-leukemia (GvL) or graft- versus-tumor (GvT) efficacy of the allo HSCT.

In some embodiments, the hematological cancer is a myeloid leukemia.

The poly-donor CD4^{IL-10} cells may target and kill cancer cells that express CD13. The poly-donor CD4^{IL-10} cells may target and kill cancer cellsthat express HLA-class 1. The mycloid leukemia may be acute myeloid leukemia (AML).

The allo-HS CT graft may be obtained from a related or unrelated donorwith respect to the recipient. The poly-donor CD4^{IL-10} cells may be non- autologous to the recipient. The poly-donor CD4^{IL-10} cells may be allogeneic to the recipient. In some instances, the poly-donor CD4^{IL-10} cells are not anergized to hostallo-antigens prior to administration to the host.

The poly-donor CD4^{IL-10} cells may be Tr1-like cells.

The poly-donor CD4^{IL-10} cells may be polyclonal. The poly-donor CD4^{IL-10} cells may be polyclonal and non-autologous to the recipient.

The poly-donor CD4^{IL-10} cells may be isolated from at least three donors prior to being genetically modified. In some instances, none of the at least three donors is the same donor as the allo-HSCT donor. The allo-HSCT graft may be obtained from a matched or mismatched donor with respect to the recipient.

The poly-donor CD4^{IL-10} cells may target and kill cells that express CD54. The poly-donor CD4^{IL-10} cells may target and kill cancer cells that express HLA-class I and CD54. The poly-donor CD4^{IL-10} cells may target and kill cancer cells that express CD112. The poly-donor CD4^{IL-10} cells may target and kill cancer cells that express CD58. The poly-donor CD4^{IL-10} cells may target and kill cancer cells in the host.

Also provided is a population of CD4⁺ cells as defined in the claims for use in a method of treating a hematological cancerby allogeneic hematopoietic stem cell transplant (allo-HSCT), the method comprising:
administering allo-HSCT graft to a subject (host);
administering to the allo-HSCT recipient (host) an amount of poly-donor CD4^{IL-10} cells sufficient to suppress graft-versus-host disease (GvHD) without suppressing graft-versus-leukemia (GvL) or graft-versus-tumor (GvT) efficacy of the allo-HSCT graft;
wherein the poly-donor CD4^{IL-10} cells comprise CD4⁺ T cells obtained from at least three different T cell donors and genetically modified by vector-mediated gene transfer of the coding sequence of human IL-10 under control of a constitutive promoter;
wherein the poly-donor CD4^{IL-10} cells are non-autologous to the recipient and non-autologous to the allo-HSCT donor;
wherein the poly-donor CD4^{IL-10} cells are not anergized to host allo-antigens prior to administration to the host; and
wherein the poly-donor CD4^{IL-10} cells are polyclonal and Tr1-like.

Also provided is a population of CD4⁺ cells as defined in the claims for use in a method of treating a hematological cancer by allogeneic hematopoietic stem cell transplant (allo-HSCT), the method comprising:
administering allo-HSCT graft to a subject (host);
administering to the allo-HSCT recipient (host) an amount of poly-donor CD4^{IL-10} cells sufficient to suppress graft-versus-host disease (GvHD) without suppressing graft-versus-leukemia (GvL) or graft-versus-tumor (GvT) efficacy of the allo-HSCT graft;
wherein the poly-donor CD4^{IL-10} cells comprise CD4⁺ T cells obtained from at least three different T cell donors and genetically modified by vector-mediated gene transfer of the coding sequence of human IL-10 under control of a constitutive promoter;
wherein the poly-donor CD4^{IL-10} cells target and kill cancer cells in the host;
wherein the poly-donor CD4^{IL-10} cells are not anergized to host allo-antigens prior to administration to the host; and
wherein the poly-donor CD4^{IL-10} cells are non-autologous to the recipient, and polyclonal, and are Tr1-like.

### 5. BRIEF DESCRIPTION OF THE DRAWINGS

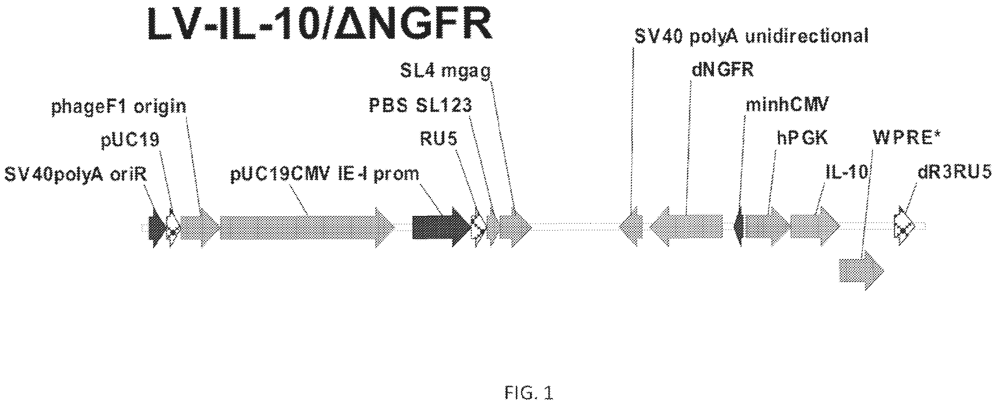

**FIG. 1** illustrates the partial structure of a bidirectional lentiviral vector for delivering human IL-10 and ΔNGFR coding sequences into CD4⁺ T cells from multiple donors to produce poly-donor CD4^{IL-10} cells.

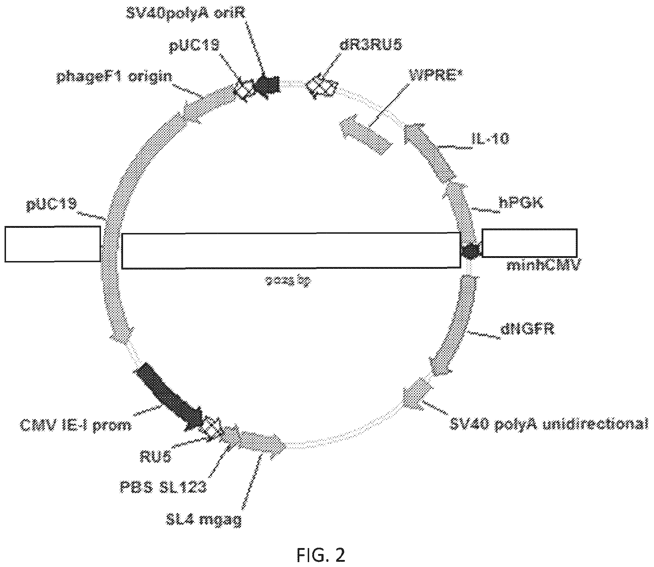

**FIG. 2** illustrates the complete and circular structure of a bidirectional lentiviral vector (hPCrK_IL10.WPRF.mhCMV. ΔNGFR.SV40PA) for delivering human IL-10 and ΔNGFR coding sequences into CD4⁺ T cells from multiple donors to produce poly-donor CD4^{IL-10} cells.

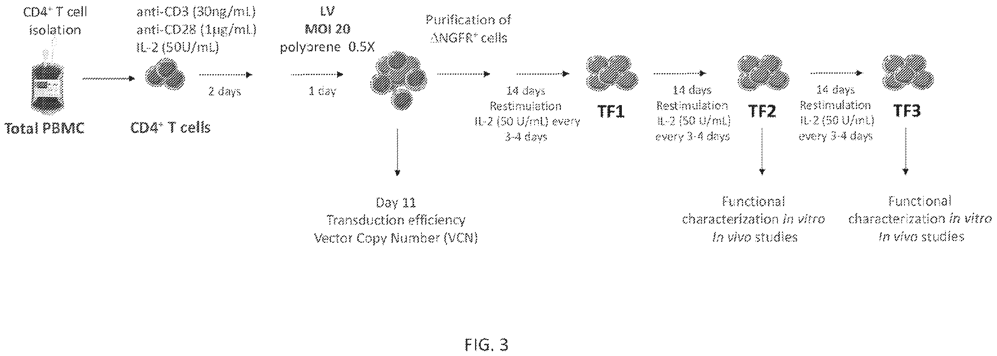

**FIG. 3** illustrates an exemplary protocol for generating CD4^{IL-10} cells.

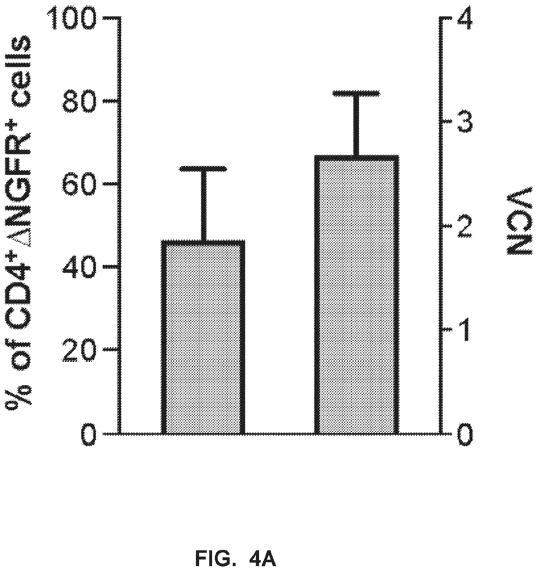

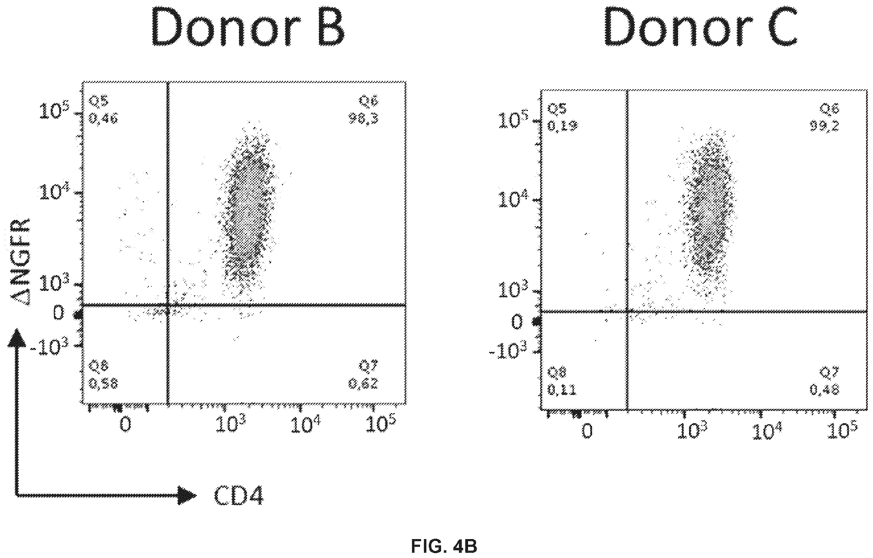

**FIG. 4A** shows percentages of CD4⁺ΔNGFR⁺ cells (mean± SD, n=10 grey bar) and vector copy numbers (VCN, mean ± SD, n=10 orange bar) in human CD4⁺ T cells transduced with LV-IL-10/ΔNGFR (a bidirectional lentiviral vector encoding for human IL-10 and a truncated form the human NGF receptor). **FIG. 4B** shows FACS analysis of expression of CD4 and ΔNGFR in human CD4⁺ T cells from two representative donors (Donor B and Donor C) transduced with LV-IL-10/ΔNGFR and purified using anti-CD271 Microbeads.

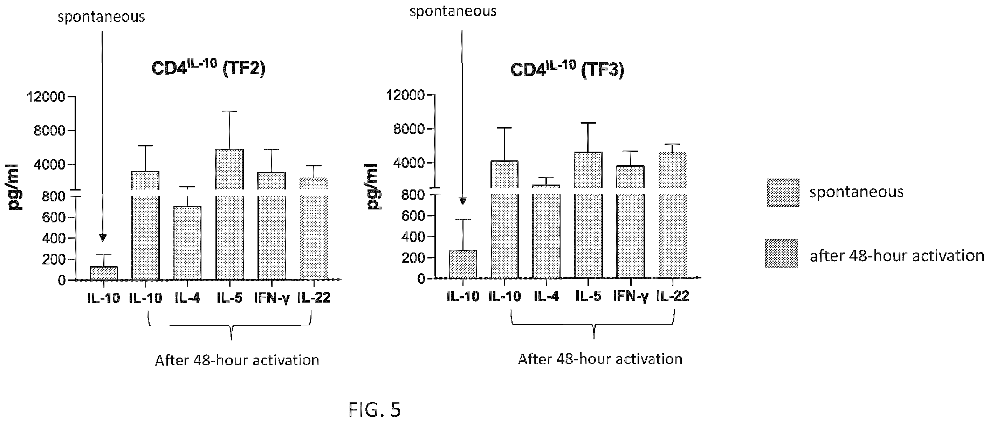

**FIG. 5** shows cytokine production profile of single donor CD4^{IL-10} cells after the second (TF2) and third (TF3) restimulation. The TF2 and TF3 CD4^{IL-10} cells were left unstimulated (orange bar) or stimulated with immobilized anti-CD3 and soluble anti-CD28 mAbs (grey bars) for 48hrs. Culture supernatants were collected and levels of IL-10, IL-4, IL-5, IFN-γ and IL-22 were determined by ELISA. All samples were tested in triplicate. Mean± SD, n=8 donors tested are presented.

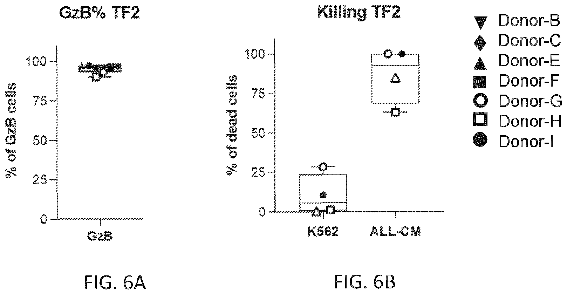

**FIG. 6A** shows the percentage of CD4^{IL-10} cells expressing granzyme B (GzB) after 2^{nd} round of stimulation (TF2) analyzed by FACS. Box and whiskers of n=7 donors and single donors are presented. **FIG. 6B** shows % dead cells when CD4^{IL-10} cells (10⁵/well) were co-cultured with K562 and ALL-CM cells (10⁵/well) at 1:1 ratio for 3 days. Box and whiskers represent data from n=4 donors and dots represent data from single donors.

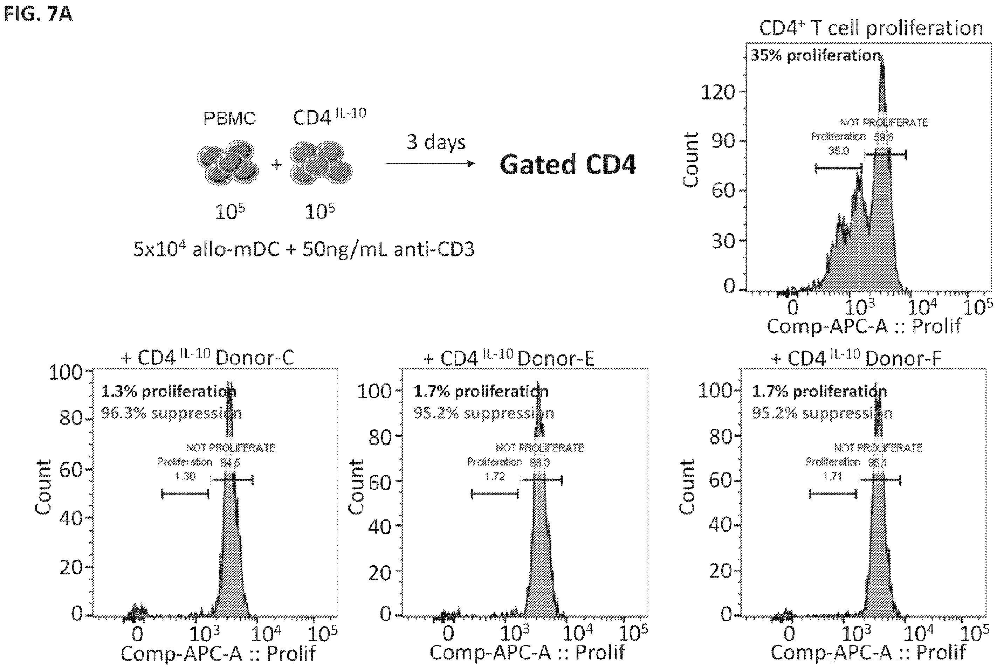

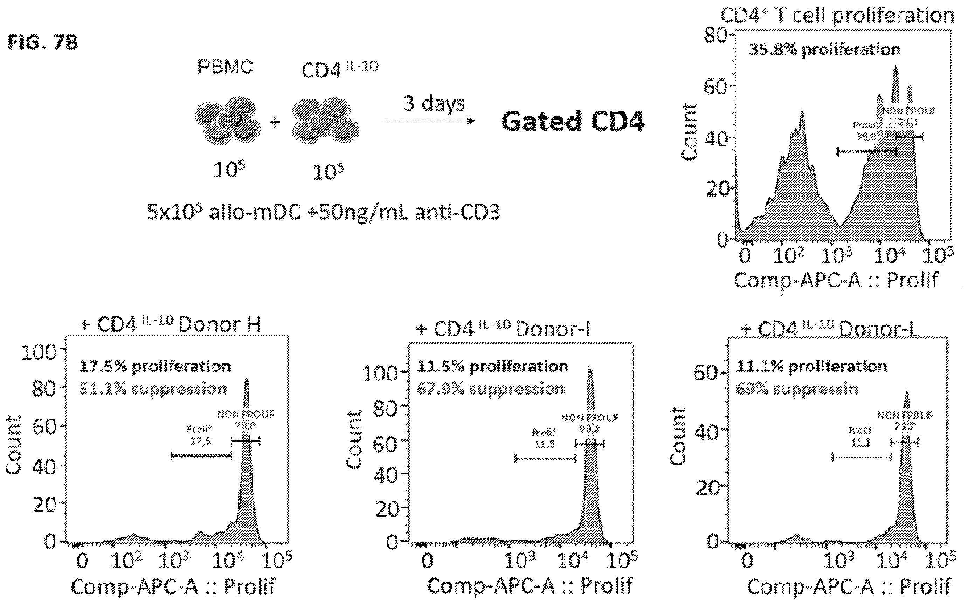

**FIGs. 7A** **and** **7B** show that single donor CD4^{IL-10} cells can suppress the proliferation of allogeneic CD4⁺ T cells. Allogeneic PBMC cells were labeled with eFluor^{®} 670 (10⁵ cells/well) and stimulated with allogenic mature dendritic (DC) cells (5×10⁴ cells/well) and soluble anti-CD3 mAbs in the absence or presence of CD4^{IL-10} cells (10⁵ cells/well) at a 1:1 Responder: Suppressor ratio. After 4 days of culture, the percentages of proliferating responder cells were determined by eFluor^{®} 670 dilution with flow cytometry after gating on CD4⁺ΔNGFR⁻ T cells. FIG. 7A show results from Donor-C, Donor-E, and Donor-F and FIG. 7B show results from Donor-H, Donor-I and Donor-L. Percentages of proliferation and suppression are indicated. The suppression mediated by CD4^{IL-10} cells was calculated as follows: 100-([proliferation of responders in the presence of CD4^{IL-10} cells/proliferation of responders alone]x 100).

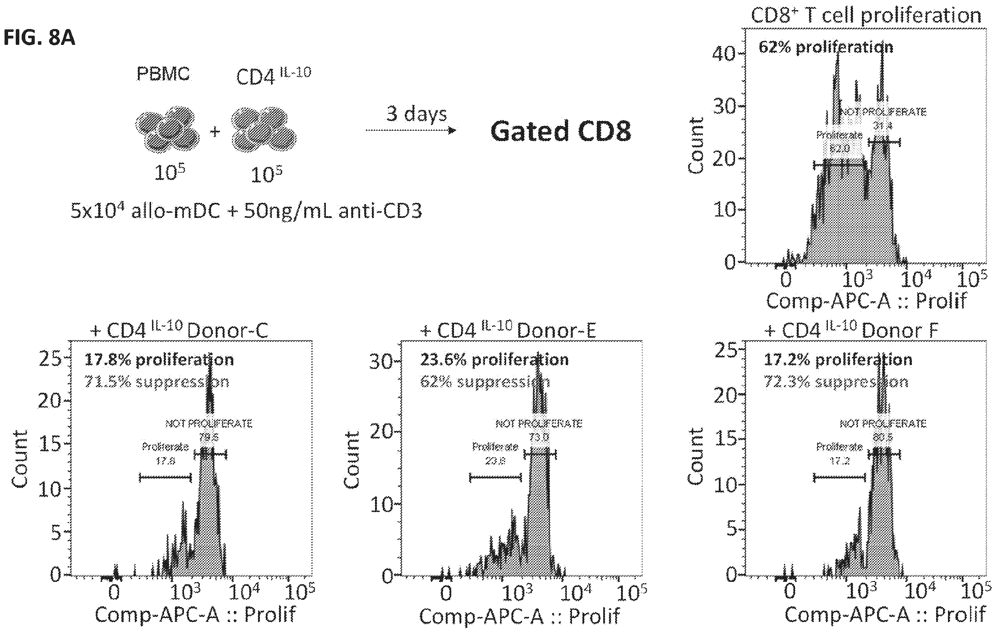

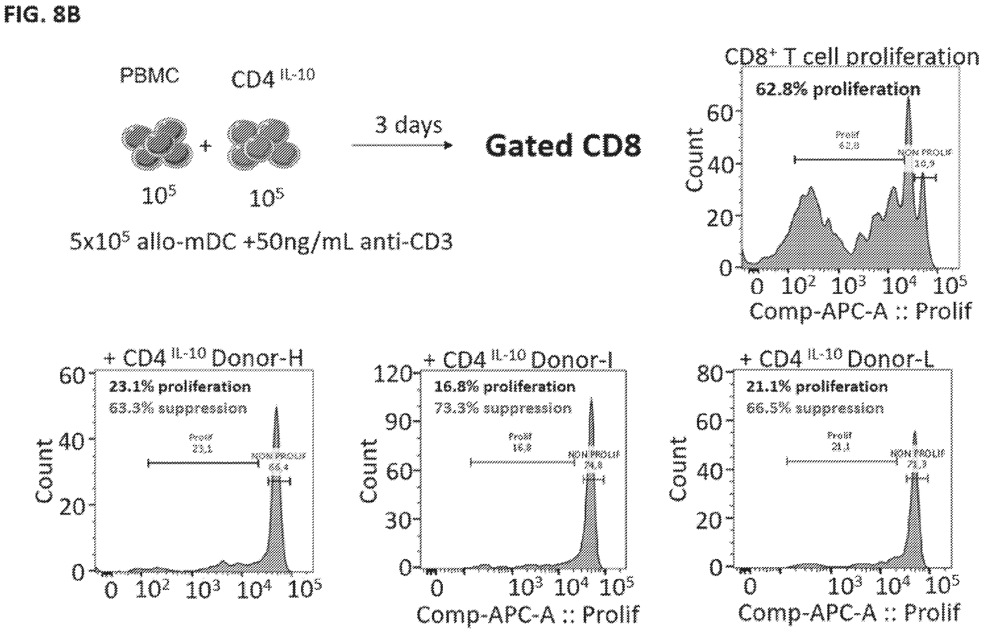

**FIGs. 8A** **and** **8B** show that single donor CD4^{IL-10} cells can suppress the proliferation of allogeneic CD8⁺ T cells. Allogeneic PBMC cells were labeled with eFluor^{®} 670 (10⁵ cells/well) and stimulated with allogeneic mature dendritic (DC) cells (5×10⁴ cells/well) and soluble anti-CD3 mAbs in the absence or presence of CD4^{IL-10} cells (10⁵ cells/well) at a 1:1 Responder: Suppressor ratio. After 4 days of culture, the percentages of proliferating responder cells were determined by eFluor^{®} 670 dilution with flow cytometry after gating on CD4⁺ΔNGFR⁻ T cells. FIG. 8A show results from Donor-C, Donor-E, and Donor-F and FIG. 8B show results from Donor-H, Donor-I and Donor-L. Percentages of proliferation and suppression are indicated. The suppression mediated by CD4^{IL-10} cells was calculated as follows: 100-([proliferation of responders in the presence of CD4^{IL-10} cells/proliferation of responders alone]x 100).

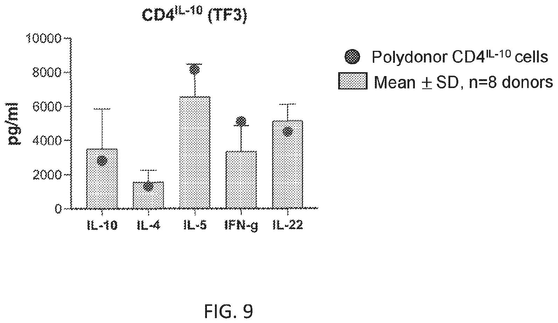

**FIG. 9** shows cytokine production profile of poly-donor CD4^{IL-10} cells after third (TF3) restimulation, compared to mean levels (+/- SD) produced by CD4^{IL-10} cells from 8 individual donors. The TF3 CD4^{IL-10} cells from three donors were pooled at a 1:1:1 ratio and stimulated with immobilized anti-CD3 and soluble anti-CD28 mAbs for 48hrs. Culture supernatants were collected and levels of IL-10, IL-4, IL-5, IFN-γ and IL-22 were determined by ELISA. Dots are results of polydonor CD4^{IL-10} cells; gray bars represent mean± SD, n=8 single donors.

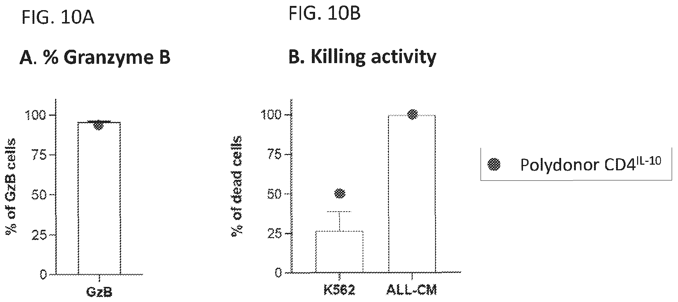

**FIG. 10A** shows the percentage of poly-donor CD4^{IL-10} cells expressing granzyme B (GzB) compared to mean % levels (+/- SD) of granzyme B expression by CD4^{IL-10} cells of n=3 single donors used to generate the pool. Cells were analyzed by FACS after the 3^{rd} round of stimulation (TF3). FIG. 10B shows % dead cells when poly-donor CD4^{IL-10} cells (10⁵/well) were co-cultured with K562 and ALL-CM cells (10⁵/well) at 1:1 ratio for 3 days. Residual leukemic cells (CD45'CD33') were counted by FACS for each target cell. Dots are results of poly-donor CD4^{IL-10} and gray bars represent meant SD of n=3 single donors used to generate the pool.

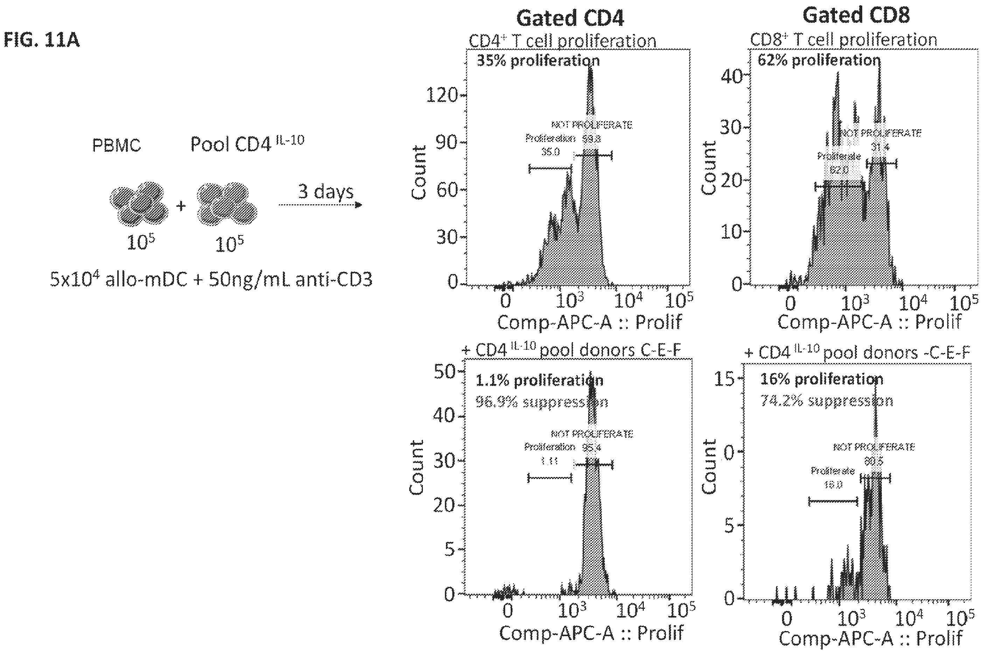

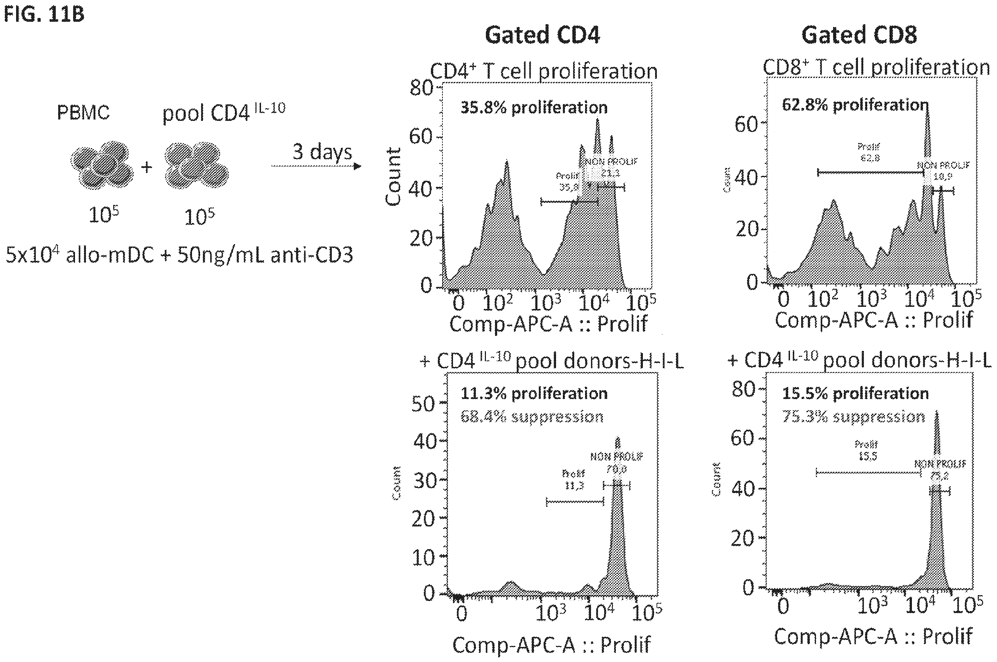

**FIGs. 11A** **and** **11B** show that poly-donor CD4^{IL-10} cells can suppress the proliferation of allogeneic CD4' T cells and CD8' T cells. Allogeneic PBMC cells were labeled with eFluor^{®} 670 (10⁵ cells/well) and stimulated with allogeneic mature dendritic (DC) cells (5×10⁴ cells/well) and soluble anti-CD3 mAbs in the absence or presence of poly-donor CD4^{IL-10} cells (10⁵ cells/well) at a 1:1 Responder: Suppressor ratio. After 4 days of culture, the percentages of proliferating responder cells were determined by eFluor^{®} 670 dilution with flow cytometry after gating on CD4⁺ΔNGFR⁻T cells and CD8⁺ΔNGFR⁻T cells. FIG. 11A shows results from poly-donor CD4^{IL-10} cells containing CD4⁺ cells pooled from Donor-C, Donor-E, and Donor-F. FIG. 11B shows results from poly-donor CD4^{IL-10} cells containing CD4⁺ cells pooled from Donor-H, Donor-I, and Donor-L. The suppression mediated by CD4^{IL-10} cells was calculated as follows: 100-([proliferation of responders in the presence of CD4^{IL-10} cells/proliferation of responders alone]x 100).

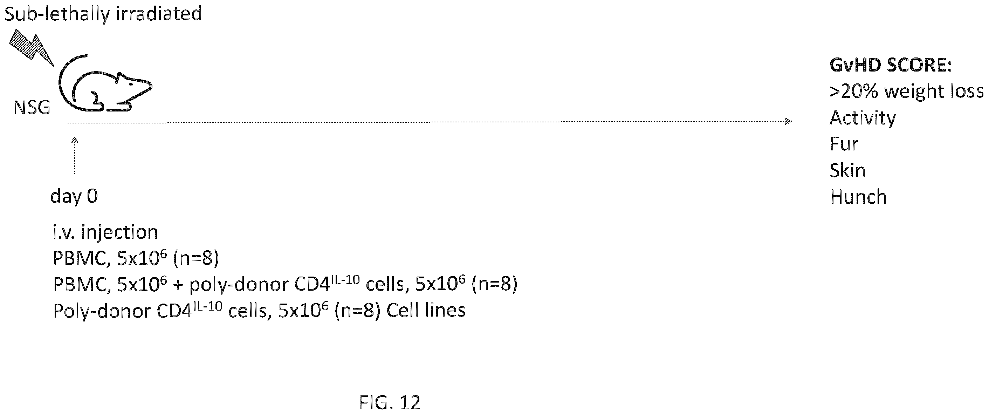

**FIG. 12** illustrates a protocol for testing induction of GvHD by human PBMC and/or poly-donor CD4^{IL-10} cells injected on day 0 post-irradiation.

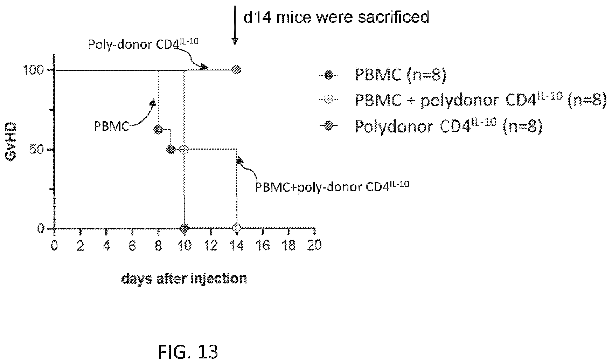

**FIG. 13** shows % of NSG mice demonstrating GvHD in each day after injection of PBMC (5×10⁶ cells/mouse), poly-donor (three donors) CD4^{IL-10} cells (5×10⁶ cells/mouse), or PBMC (5×10⁶ cells/mouse) in combination with poly-donor CD4^{IL-10} cells (three donors) (5×10⁶ cells/mouse).

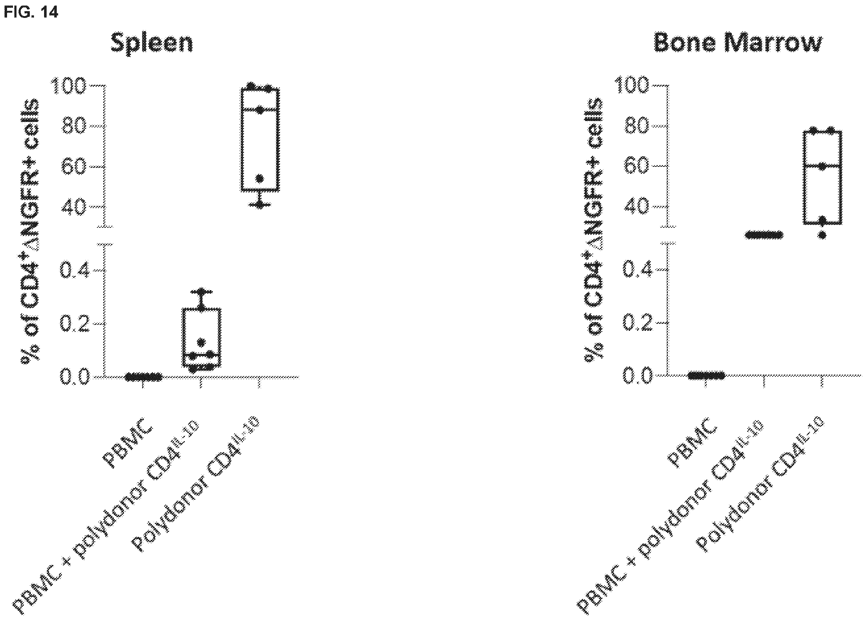

**FIG. 14** shows migration of CD4^{IL-10} cells to spleen and bone marrow in NSG mice injected with PBMC (5×10⁶ cells/mouse), poly-donor (three donors) CD4^{IL-10} cells (5×10⁶ cells/mouse), or PBMC (5×10⁶ cells/mouse) in combination with poly-donor CD4^{IL-10} cells (three donors) (5×10⁶ cells/mouse). Box and whiskers on n=8 donors and single donors are presented.

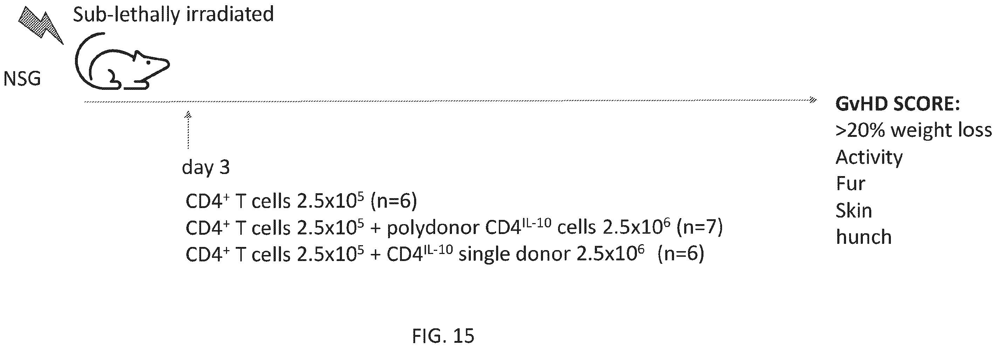

**FIG. 15** illustrates a protocol for testing induction of GvHD by CD4+ T cells and poly-donor or single-donor CD4^{IL-10} cells injected on day 0 post-irradiation.

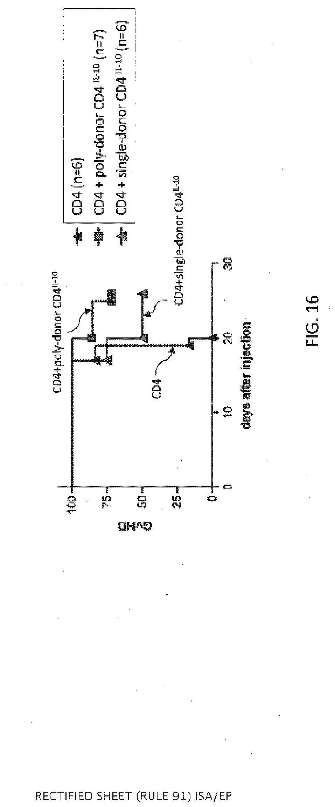

**FIG. 16** shows % of NSG mice demonstrating GvHD on each day after injection.

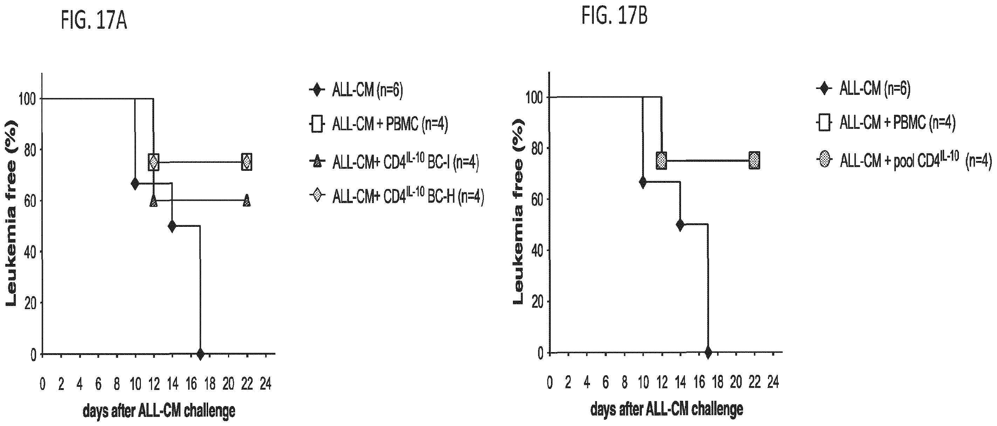

**FIG. 17A and 17B** shows graft-versus-leukemia (GvL) effect tested based on reduction of circulating leukemia cells and long-term leukemia free survival. Leukemia was measured as previously described (Locafaro G. et al Molecular Therapy 2017). NSG mice were sub-lethally irradiated and intravenously injected with myeloid leukemia cells (ALL-CM) (5×10⁶) at day 0. **FIG. 17A** shows leukemia free survival rate in the animals injected with PBMC (5×10⁶) or single donor (from donor BC-I and donor BC-H) CD4^{IL-10} cells (2.5×10⁶) at day 3. **FIG. 17B**. shows leukemia free survival rate in the animals injected with PBMC (5×10⁶) or poly-donor CD4^{IL-10} cells (2.5×10⁶) at day 3.

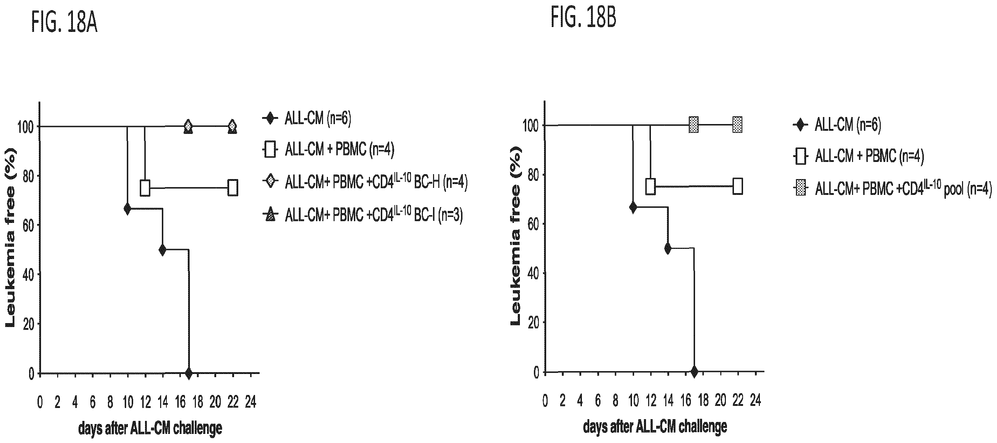

**FIG. 18A and FIG. 18B** show long-term leukemia free survival rate measured in NSG mice sub-lethally irradiated and intravenously injected with ALL-CM cells (5×10⁶) at day 0. **FIG. 18A** shows data from animals injected with mononuclear cells (PBMC) (5×10⁶) alone or mononuclear cells (PBMC) (5×10⁶) + single donor (from donor BC-H and donor BC-I) CD4^{IL-10} cells (2.5×10⁶) at day 3. **FIG. 18B** shows data from animals injected with mononuclear cells (PBMC) (5×10⁶) alone or mononuclear cells (PBMC) (5×10⁶) + poly-donor CD4^{IL-10} cells (2.5×10⁶) at day 3.

The figures depict various embodiments of the present invention for purposes of illustration only.

### 6. DETAILED DESCRIPTION

### 6.1. Definitions

Unless defined otherwise, all technical and scientific terms used herein have the meaning commonly understood by a person skilled in the art to which this invention belongs. As used herein, the following terms have the meanings ascribed to them below.

**"Graft-versus-leukemia effect"** or **"GvL"** refers to an effect that appears after allogeneic hematopoietic stem cell transplantation (HSCT) or bone marrow transplantation (BMT). T lymphocytes in the allogeneic graft eliminate malignant residual host leukemia cells.

**"Graft versus tumor effect"** or **"GvT** refers to an effect that appears after allogeneic hematopoietic stem cell transplantation (HSCT) or bone marrow transplantation (BMT). T lymphocytes in the allogeneic graft eliminate malignant residual host cancer cells, e.g., cells of myeloma and lymphoid and myeloid leukemias, lymphoma, multiple myeloma and possibly breast cancer. The term GvT is generic to GvL.

The terms **"treatment", "treating",** and the like are used herein in the broadest sense understood in the medical arts. In particular, the terms generally mean obtaining a desired pharmacologic and/or physiologic effect. The effect may be prophylactic, in terms of completely or partially preventing a disease, condition, or symptoms thereof, and/or may be therapeutic in terms of a partial or complete cure for a disease or condition and/or adverse effect, such as a symptom, attributable to the disease or condition. "Treatment" as used herein covers any treatment of a disease or condition of a mammal, particularly a human, and includes: (a) preventing the disease or condition from occurring in a subject which may be predisposed to the disease or condition but has not yet been diagnosed as having it; (b) inhibiting the disease or condition (e.g., arresting its development); or (c) relieving the disease or condition (e.g., causing regression of the disease or condition, providing improvement in one or more symptoms). Improvements in any conditions can be readily assessed according to standard methods and techniques known in the art. The population of subjects treated by the method of the disease includes subjects suffering from the undesirable condition or disease, as well as subjects at risk for development of the condition or disease.

**"HLA-matched"** as used herein refers to a pair of individuals having a matching HLA allele in the HLA class I (HLA-A, HLA-B, and HLA-C) and class II (HLA-DRB1 and HLA-DQB1) loci that allow the individuals to be immunologically compatible with each other. HLA compatibility can be determined using any of the methods available in the art, for example, as described in Tiervy, Haematologica 2016 Volume 101(6):680-687.

For a given locus, a pair of individuals have 2/2 match when each of two alleles of one individual match with the two alleles of the other individual. A pair of individuals have ½ match when only one of two alleles of one individual match with one of two alleles of the other individual. A pair of individuals have 10/10 match at the HLA-A, HLA-B, HLA-C, HLA-DRB1, and HLA-DQB1 loci when all of the ten alleles (two for each of the HLA-A, HLA-B, HLA-C, HLA-DRB1, and HLA-DQB1 loci) of one individual match with all ten alleles of the other individual.

In preferred embodiments, allele level typing is used for determination of HLA compatibility. Allele level typing corresponds to a unique nucleotide sequence for an HLA gene, as defined by using all digits in the first, second, third and fourth fields, e.g. A*02:01:01:01. Functionally, the third and fourth fields which characterize alleles that differ, respectively, by silent substitutions in the coding sequence and by substitutions in the non-coding sequence, are irrelevant, except when substitutions prevent the expression of HLA alleles (*e.g.* the null allele B*15:01:01:02N). Missing a null allele will lead to a mismatch that is very likely to be recognized by alloreactive T cells and have a deleterious clinical impact. Substitutions in non-coding sequences may influence the level of expression (e.g. the A24low allele A*24:02:01:02L). Such variability may also have an impact on anti-HLA allorecognition.

The term **"HLA-mismatched"** as used herein refers to a pair of individuals having a mis-matching HLA allele in the HLA class I (HLA-A, HLA-B, and HLA-C) and class II (HLA-DRB1 and HLA-DQB1) loci that make the individuals to be immunologically incompatible with each other.

The term **"partially HLA-mismatched"** as used herein refers to a pair of individuals having a mis-matching HLA allele in the HLA class I (HLA-A, HLA-B, and HLA-C) and class II (HLA-DRB1 and HLA-DQB1) loci that make the individuals to be immunologically incompatible with each other in a permissible degree. Some studies have identified permissive mismatches. Some HLA class I incompatibilities are considered to be more permissive.

**"HLA haplotype"** refers to a series of HLA loci-alleles by chromosome, one passed from the mother and one from the father. Genotypes for HLA class 1 (HLA-A, HLA-B, and HLA-C) and class II (HLA-DRB1 and HLA-DQB1) loci can be used to determine the HLA haplotype.

The term **"therapeutically effective amount"** is an amount that is effective to treat, and thus ameliorate a symptom of a disease. A therapeutically effective amount can be a **"prophylactically effective amount"** as prophylaxis can be considered therapy.

The term **"ameliorating"** refers to any therapeutically beneficial result in the treatment of a disease state, e.g., a neurodegenerative disease state, including prophylaxis, lessening in the severity or progression, remission, or cure thereof.

### 6.2. Other interpretational conventions

Ranges recited herein are understood to be shorthand for all of the values within the range, inclusive of the recited endpoints. For example, a range of 1 to 50 is understood to include any number, combination of numbers, or sub-range from the group consisting of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, and 50.

### 6.3. Summary of experimental observations

The present disclosure provides the methods for production and use of highly purified, allogeneic CD4⁺ T cells that have been transduced with a bidirectional lentiviral vector containing the human IL-10 gene and a truncated, non-signaling form of the human NGFR. The successfully transduced CD4⁺ T cells were purified utilizing a NGFR specific monoclonal antibody resulting in > 95% pure IL-10 producing and NGFR expressing CD4⁺ T cells (designated CD4^{IL-10} cells). CD4^{IL-10} cells from 3 different allogeneic HLA mismatched donors were pooled at 1:1:1 ratios.

These pooled populations, also referred to herein as poly-donor CD4^{IL-10} cells, had cytokine production profiles comparable to those of single-donor CD4^{IL-10} cells and naturally derived type 1 regulatory T (Tr1) cells. They produce high levels of IL-10, IL-22, IFN-y, IL-5 and low levels of IL-4. The poly-donor CD4^{IL-10} cells were polyclonal (has multiple antigen specificities) and suppressed proliferation of both allogencic CD4⁺ and CD8⁺ T cells *in vitro.* In addition, they specifically killed myeloid leukemia cells *in vitro.* Adoptive transfer of poly-donor CD4^{IL-10} cells in a humanized mouse model for Graft versus Host Disease (GvHD) indicated that these cells efficiently home to the spleen. Adoptive transfer of poly-donor CD4^{IL-10} cells in a humanized mouse model of GvHD inhibited severe xeno-GvHD induced by human CD4⁺ T cells. Importantly, even at high concentrations, poly-donor CD4IL-10 cells did not induce GvHD by themselves. These results demonstrate that poly-donor CD4^{IL-10} cells can be used for the treatment and/or prevention of GvHD; can be used as an adjunct to allogeneic hematopoietic stem cell transplant (HSCT) for treatment of leukemias and other malignancies to reduce GvHD while preserving GvL or GvT therapeutic effects of the HSCT; and for treating cell and organ rejection and autoimmune and inflammatory diseases.

### 6.4. Poly-donor CD4^{IL-10} cells

The invention provides a population of CD4⁺ T cells that have been genetically modified to comprise an exogenous polynucleotide encoding IL-10 (CD4^{IL-10} cells). The population comprises CD4⁺ T cells obtained from at least three different T cell donors (poly-donor CD4^{IL-10} cells) and the exogenous polynucleotide is integrated into the T cell nuclear genome.

### 6.4.1. CD4⁺ T cells and T cell donors

CD4⁺ T cells used in poly-donor CD4^{IL-10} populations can be isolated from peripheral blood, cord blood, or other blood samples from a donor, using methods available in the art. Typically, CD4⁺ T cells are isolated from peripheral blood. CD4⁺ T cells may be isolated from peripheral blood obtained from third party-bloodbanks.

CD4⁺ T cells may be isolated from a prior-frozen stock of blood or a prior-frozen stock of peripheral blood mononuclear cells (PBMCs). CD4⁺ T cells may be isolated from peripheral blood or from PBMCs that have not previously been frozen. The CD4+ T cells may be separately isolated from blood or PBMCs obtained from a plurality of donors, and then pooled. The CD4+ T cells may be isolated from blood or PBMCs that have first been pooled from a plurality of donors.

The CD4⁺ T cells may be obtained from three, four, five, six,seven, eight, nine, or ten different T cell donors.

The at least three different T cell donors may be selected without regard to genotype. The at least three different T cell donors may be selected based on genotype.

The at least three different T cell donors may be selected based on their HLA haplotypes.

Some or all of the at least three different T cell donors may have matching HLA haplotypes. Some or all of the at least three different T cell donors may have a mis-matched HLA haplotype.

In some embodiments, all of the CD4⁺ T cells in the population have at least 5/10, 6/10, 7/10, 8/10, or 9/10 match at the HLA-A, HLA-B, HLA-C, HLA-DRB1, and HLA-DQB1 loci to each other. In some embodiments, all of the CD4⁺ T cells in the population have at least 4/8, 5/8, 6/8, 7/8, or 8/8 match at the HLA-A, HLA-B, HLA-C, and HLA-DRB1 loci to each other. In some embodiments, all the CD4⁺ T cells in the population have 2/2 match at the HLA-A locus to each other. In some embodiments, all the CD4⁺ T cells in the population have 2/2 match at the HLA-B locus to each other. In some embodiments, all the CD4⁺ T cells in the population have 2/2 match at the HLA-C locus to each other. In some embodiments, all the CD4⁺ T cells in the population have at least 3/4 or 4/4 match at the HLA-DRB1 and HLA DQB1 loci with each other. In some embodiments, all the CD4⁺ T cells in the population have an A*02 allele.

In some instances, none of the at least three different T cell donors is a host to be treated with the CD4^{IL-10} cells. In some instances, none of the at least three different T cell donors is a donor of stem cells (e.g., HSC), tissue or organ that will be used together with the CD4^{IL-10} cells in the methods of treatment described herein.

One or more of the T cell donors may be HLA-mismatched or partially HLA-mismatched to the patient to be treated (host). One or moreof the T cell donors may have less than 5/10, 6/10, 7/10, 8/10, 9/10 or 10/10 match at the HLA-A, HLA-B, HLA-C, HLA-DRB1, and HLA-DQB 1 loci to the patient. One or more of the T cell donors may have less than 4/8, 5/8, 6/8, 7/8, or 8/8 match at the HLA-A, HLA- B, HLA-C, and HLA-DRB1 loci to the patient. One or more of the T cell donors may have less than 2/2 match at the HLA-A, HLA-B, or HLA-C locus to the patient. One or more of the T cell donors may have less than 2/4, 3/4 or 4/4 match at the HLA- DRB1 and HLA-DQB1 loci to the patient.

One or more of the T cell donors may be HLA-mismatched or partially HLA-mismatched with the HSC donor. One or more of the T cell donors may have less than 5/10, 6/10, 7/10, 8/10, 9/10 or 10/10 match at the HLA-A, HLA-B,HLA-C, HLA-DRB1, and HLA-DQB1 loci to the HSC donor. One or more of the T cell donors may have less than 4/8, 5/8, 6/8, 7/8, or 8/8 match at the HLA-A, HLA-B, HLA-C, and HLA-DRB1 loci to the HSC donor. One or more of the T cell donors may have less than 2/2 match at the HLA-A, HLA-B, or HLA-C locus to the HSC donor. One or more of the T cell donors may have less than 3/4 or 4/4 match at theHLA-DRB1 and HLA-DQB1 loci to the HSC donor.

In some instances, none of the CD4⁺ T cells is immortalized.

### 6.4.2. Exogenous polynucleotide encoding IL-10

Poly-donor CD4^{IL-10} cells of the invention are CD4⁺ T cells that have been genetically modified to comprise an exogenous polynucleotide encoding IL-10 wherein the exogenous polynucleotide is integrated into the T cell nuclear genome. The exogenous polynucleotide may comprise an IL-10-encoding polynucleotide segment operably linked to expression control elements.

The IL-10-encoding polynucleotide segment can encode IL-10 of a human, bonobo or rhesus. The IL-10-encoding polynucleotide segment may encode human IL-10 having the sequence of SEQ ID NO: 1. The IL-10-encoding polynucleotide segment may encode a variant of human IL-10 having at least 90%, 95%, 98%, or 99% sequence identity to SEQ ID NO: 1. The IL-10-encoding polynucleotide segment may have the nucleotide sequence of SEQ ID NO:2. The IL-10-encoding polynucleotide segment may have at least 90%, 95%, 98%, or 99% sequence identity to SEQ ID NO: 2.

The exogenous polynucleotide may encode viral-IL-10. The exogenous polypeptide may encode IL-10 from HCMV, GMCMV, RhCMV, BaCMV, MOCMV, SMCMV, EBV, Bonobo-HV, BaLCV, OvHV-2, EHV-2, CyHV-3, AngHV-1, ORFV, BPSV, PCPV, LSDV, SPV, GPV, or CNPV. The exogenous polypeptide may encode viral IL-10 from EBV or ORFV.

The exogenous polynucleotide may further comprise expression control elements that direct expression of the encoded IL-10 in transduced CD4⁺ T cells.

The expression control elements may comprise a promoter capable of directing expression of IL-10 in CD4⁺ T cells. The promoter may drive constitutive expression of IL-10 in CD4⁺ T cells. The promoter may drive expression of IL-10 in activated CD4⁺ T cells.

An inducible promoter may be used to induce expression of IL-10 when therapeutically appropriate. The IL-10 promoter may be used. A tissue-specific promoter may be used. A lineage-specific promoter may be used. A ubiquitously expressed promoter may be used.

A native human promoter may be used. A human elongation factor (EF)1α promoter may be used. A human phosphoglycerate kinase promoter (PGK) may be used. A human ubiquitin C promoter (UBI-C) may be used.

A synthetic promoter may be used. A minimal CMV core promoter may be used. An inducible or constitutive bidirectional promoter may be used. The synthetic bidirectional promoter disclosed in Amendola et al., Nature Biotechnology, 23(1):108-116

(2005) may be used. This promoter can mediate coordinated transcription of two mRNAs in a ubiquitous or a tissue- specific manner. The bidirectional promoter may induce expression of IL-10and a selection marker.

In some embodiments, the exogenous polynucleotide further comprises a segment encoding a selection marker that permits selection of successfully transduced CD4⁺ T cells. In some embodiments, the selection marker is ΔNGFR. The selection marker may be a polypeptide having the sequence of SEQ ID NO:3. The selection marker may be a polypeptide having at least 90%, 95%, 98%, or 99% sequence identity to SEQ ID NO: 3. The nucleotide sequence encoding the ΔNGFR selection marker may have the sequence of SEQ ID NO: 4. The nucleotide sequence encoding the ΔNGFR selection marker may have at least 90%, 95%, 98%, or 99% sequence identity to SEQ ID NO: 4.

The selection marker may be a truncated form of EGFR polypeptide. The selection marker may be a truncated form of the human EGFRpolypeptide, optionally huEGFRt disclosed in Wang et al. "A transgene-encoded cell surface polypeptide for selection, in vivo tracking, and ablation of engineered cells", Blood, v. 118, n. 5 (2011).

The exogenous polynucleotide may further comprise a sequence encoding an antibiotic resistance gene. The exogenous polynucleotide may comprise a sequence encoding an ampicillin resistance gene. The exogenous polynucleotide may comprise a sequence encoding a kanamycin resistance gene.

The exogenous polynucleotide may be delivered into CD4+ Tcells using a vector. The vector may be a plasmid vector. The vector may be a viral vector.

The exogenous polynucleotide may be delivered into CD4+ Tcells using a lentiviral vector and the exogenous polynucleotide may comprise lentiviral vector sequences. A lentiviral vector disclosed in Mátrai et al., Molecular Therapy 18(3):477-490 (2010) ("Mátrai") may be used.

The lentiviral vector may be capable of integrating into the T cell nuclear genome. In some instances, the lentiviral vector is not capable of integrating into T cell nuclear genome. An integration-deficient lentiviral vector may be used. For example, an integration-deficient or other lentiviral vector disclosed in Matrai may be used. An integrase-defective lentivirus may be used. For example, an integrase-defective lentivirus containing an inactivating mutation in the integrase (D64V) can be used as described in Matrai et al., Hepatology 53:1696-1707 (2011).

In the CD4⁺ T cells of the invention, the exogenous polynucleotide is integrated in the T cell nuclear genome.

The exogenous polynucleotide may have the sequence of SEQ ID NO:5. The exogenous polynucleotide may have at least 90%, 95%, 98%, or 99% sequence identity to SEQ ID NO: 5.

### 6.4.3. Gene expression of poly-donor CD4^{IL-10} T cells

Poly-donor CD4^{IL-10} T cells express IL-10. In some embodiments, poly-donor CD4^{IL-10} T cells constitutively express IL-10. In some embodiments, poly-donor CD4^{IL-10} T cells express IL-10 when activated.

In some embodiments, poly-donor CD4^{IL-10} T cells constitutively express at least 100 pg of IL-10 per 10⁶ of the CD4⁺ T cells/ml of culture. In some embodiments, poly-donor CD4^{IL-10} T cells constitutively express at least 200pg, 500pg, 1ng, 5ng, 10ng, or 50ng of IL-10 per 10⁶ of the CD4⁺ T cells/ml of culture.

In some embodiments, poly-donor CD4^{IL-10} T cells express at least 1ng or 2ng IL-10 per 10⁶ of the CD4⁺ T cells/ml of culture after activation with a combination of anti-CD3 and anti-CD28 antibodies, or anti-CD3 antibody and anti-CD28 antibody coated beads. In some embodiments, poly-donor CD4^{IL-10} T cells express at least 5ng, 10ng, 100ng, 200ng, or 500ng IL-10 per 10⁶ of the CD4⁺ T cells/ml of culture after activation with anti-CD3 and anti-CD28 antibodies or CD3 antibody and CD28 antibody coated beads.

The amount of IL-10 production may be determined 12 hours, 24 hours, or 48 hours after activation using various methods for protein detection and measurement, such as ELISA, spectroscopic procedures, colorimetry, amino acid analysis, radiolabeling, Edman degradation, HPLC, western blotting, etc. The amount of IL-10 production may be determined by ELISA 48 hours after activation with anti-CD3 and anti-CD28 antibodies.

Poly-donor CD4^{IL-10} T cells may express IL-10 at a level at least 5-fold higher than unmodified CD4⁺ T cells. Poly-donor CD4^{IL-10} Tcells may express IL-10 at a level at least 6, 7, 8, 9, 10, 11, 12, 13, 14, 15, 20, 30, 40, or 50-fold higher than unmodified CD4⁺ T cells.

In some embodiments, poly-donor CD4^{IL-10} T cells further express a selection marker. Poly-donor CD4^{IL-10} T cells may express a protein typically expressed in Tr1 cells. Poly-donor CD4^{IL-10} T cells may express a marker protein characteristic of Tr1 cells.

In some embodiments, poly-donor CD4^{IL-10} T cells express CD49b. In some embodiments, poly-donor CD4^{IL-10} T cells express LAG-3. In some embodiments, poly-donor CD4^{IL-10} T cells express TGF-β. In some embodiments, poly-donor CD4^{IL-10} T cells express IFNγ. In some embodiments, poly-donor CD4^{IL-10} T cells express GzB. Poly-donor CD4^{IL-10} T cells may release GzB when activated with myeloid antigen-presenting cells. In some embodiments, poly-donor CD4^{IL-10} T cells express perforin. Poly-donor CD4^{IL-10} T cells may release perforin when activated with myeloid antigen-presenting cells. In some embodiments, poly-donor CD4^{IL-10} T cells express CD18. In some embodiments, poly-donor CD4^{IL-10} T cells express CD2. In some embodiments, poly-donor CD4^{IL-10} T cells express CD226. In some embodiments, poly-donor CD4^{IL-10} T cells express IL-22. In some embodiments, poly-donor CD4^{IL-10} T cells express IL-10.

Poly-donor CD4^{IL-10} T cells may exhibit at least one phenotypicfunction of Tr1 cells. The function may be secretion of IL-10, secretion of TGF-β, and by the specific killing of myeloid antigen-presenting cells through the release of Granzyme B (GzB) and perforin.

### 6.4.4. Product by process

In typical embodiments, poly-donor CD4^{IL-10} T cells are obtained by modifying CD4⁺ T cells with an exogenous polynucleotide encoding IL-10.

The exogenous polynucleotide may be introduced to CD4⁺ Tcells by a viral vector or a plasmid vector. CD4⁺ T cells may be transduced with a lentivirus containing a coding sequence of IL-10.

Poly-donor CD4^{IL-10} T cells may be generated by (i) pooling primary CD4⁺ T cells obtained from at least three different T cell donors; and (ii) modifying the pooled CD4⁺ T cells by introducing an exogenous polynucleotide encoding IL-10. In some embodiments, poly-donor CD4^{IL-10} T cells are generated by (i) modifying primary CD4⁺ T cells which have been obtained from at least three different T cell donors, wherein each donor's CD4' T cells are separately modified by introducing an exogenous polynucleotide encoding IL-10, and then (ii) pooling the genetically modified CD4⁺ T cells.

Poly-donor CD4^{IL-10} T cells may have been cultured in the presence of proteins capable of activating CD4⁺ T cells. Poly-donor CD4^{IL-10} T cells may have been cultured in the presence of anti-CD3 antibody and anti-CD28 antibody, or anti-CD3 antibody and anti-CD28 antibody coated beads. Poly-donor CD4^{IL-10} T cells may have been cultured in the presence of anti-CD3 antibodies, anti- CD28 antibodies, and IL-2, or anti-CD3 antibody and anti-CD28 antibody coated beads and IL-2. Poly-donor CD4^{IL-10} T cells may have been cultured in the presence of T Cell TransAct^{™} from Miltenyi Biotec. Poly-donor CD4^{IL-10} T cells may have been cultured in the presence of ImmunoCult Human T Cell Activator^{™} from STEMCELL Technologies.

In some embodiments, poly-donor CD4^{IL-10} T cells are in a frozen stock.

### 6.5. Pharmaceutical compositions

Pharmaceutical compositions are also described herein. The pharmaceutical composition typically comprises the poly-donor CD4^{IL-10} T cells of the invention and a pharmaceutically acceptable carrier or diluent.

The pharmaceutical composition may be formulated for administration by any route of administration appropriate for human or veterinary medicine. The composition may be formulated for intravenous (IV) administration. The composition may be formulated for intravenous (IV) infusion. When the composition is formulated for IV administration, the pharmaceutical composition may be in the form of a parenterally acceptable aqueous solution which is pyrogen-free and has suitable pH, isotonicity and stability.

The pharmaceutically acceptable carrier or diluent may be saline, lactated Ringer's solution, or other physiologically compatible solution. In various embodiments, the pharmaceutical composition solution comprises 2-20%, preferably 5%, human serum albumin.

Unit dosage forms of the pharmaceutical composition may be provided that are adapted for administration of the pharmaceutical composition by systemic administration, in particular, for intravenous administration.

The unit dosage form may contain 10⁴ to 10¹¹ poly-donor CD4^{IL-10} T cells, 10⁴ to 10¹⁰ poly-donor CD4^{IL-10} T cells, 10⁴ to 10⁹ poly-donor CD4^{IL-10} T cells,10⁵ to 10¹⁰ poly-donor CD4^{IL-10} T cells, 10⁵ to 10⁹ poly-donor CD4^{IL-10} T cells, 10⁵ to 10⁸ poly- donor CD4^{IL-10} T cells, or 10⁵ to 10⁷ poly-donor CD4^{IL-10} T cells.

The pharmaceutical composition in the unit dosage form may be in liquid form.

### 6.6. Methods of making poly-donor CD4^{IL-10} cells

In another aspect, the present disclosure provides a method of making poly-donor CD4^{IL-10} cells.

The method may comprise the steps of: (i) pooling primary CD4⁺ T cells obtained from at least three different T cell donors; and (ii) modifying the pooledCD4⁺ T cells by introducing an exogenous polynucleotide encoding IL-10. The invention provides a method comprising the steps of: (i) modifying primary CD4⁺ T cells which have been obtained from at least three different T cell donors, wherein each donor's CD4⁺ T cells are separately modified by introducing an exogenous polynucleotide encoding IL-10; and (ii) pooling the geneticallymodified CD4⁺ T cells, thereby obtaining the poly-donor CD4^{IL-10} cells. Various methods known in the art can be used to introduce an exogenous polynucleotide encoding IL-10 to primary CD4⁺ T cells.

In some embodiments, the method further comprises the step of incubating the primary CD4⁺ T cells or genetically-modified CD4⁺ T cells in the presence of an anti-CD3 antibody and anti-CD28 antibody, or anti-CD3 antibody and anti-CD28 antibody coated beads. In some embodiments, the method further comprises the step of incubating the primary CD4⁺ T cells or genetically-modified CD4⁺ T cells in the presence of anti-CD3 antibody, anti-CD28 antibody and IL-2 or anti-CD3 antibody and anti-CD28 antibody coated beads and IL-2. The method may further comprise the step of incubating the primary CD4⁺ T cells or genetically-modified CD4⁺ T cells in the presence of a mixture of feeder cells. In some embodiments, the method further comprises the step of incubating the primary CD4⁺ T cells or genetically-modified CD4' T cells in the presence of nanopreparations of anti-CD3 antibody and anti-CD28 antibody. The incubation may be done in the presence of T Cell TransAct^{™} from Miltenyi Biotec. The incubation may be done in the presence of ImmunoCult Human T Cell Activator^{™} from STEMCELL Technologies.

In some embodiments, the incubation step is performed before introducing an exogenous polynucleotide encoding IL-10. The incubation step may be performed after (i) pooling primary CD4⁺ T cells obtained from at least three different T cell donors; but before (ii) modifying the pooled CD4⁺ T cells by introducing an exogenous polynucleotide encoding IL-10. In some embodiments, the incubation step is performed after (i)primary CD4⁺ T cells are obtained from at least three different T cell donors; but before (ii) separately modifying each donor's CD4⁺ T cells by introducing an exogenous polynucleotide encoding IL-10.

In some embodiments, the incubation step is performed after step (ii). In other words, the incubation step may be performed after (ii) modifying the pooled CD4⁺ T cells by introducing an exogenous polynucleotide encoding IL-10. In some embodiments, the incubation step is performed after (ii) separately modifying each donor's CD4⁺T cells by introducing an exogenous polynucleotide encoding IL-10, but before (iii) pooling the genetically modified CD4⁺ T cells, thereby obtaining the genetically-modified CD4⁺ T cells. Insome embodiments, the incubation step is performed after (iii) pooling the genetically modifiedCD4⁺ T cells, thereby obtaining the poly-donor CD4^{IL-10} cells.

In some embodiments, the incubation step is performed more than once. In some embodiments, the incubation step is performed both before and after genetic modification of CD4⁺ T cells.

The exogenous polynucleotide may be introduced into the primary CD4⁺ T cells using a viral vector. The viral vector may be a lentiviral vector. The exogenous polynucleotide may comprise a segment encoding IL- 10 having the sequence of SEQ ID NO: 1. The exogenous polynucleotide may comprise a segment encoding IL-10 having at least 90%, 95%, 98%, or 99% sequence identity to SEQ ID NO: 1. The IL-10-encoding polynucleotide segment may have the sequence of SEQ ID NO: 2. The IL-10-encoding polynucleotide segment may have at least 90%, 95%, 98%, or 99% sequence identity to SEQ ID NO: 2. In some embodiments,the exogenous polynucleotide further comprises a segment encoding a marker permitting selection of successfully transduced CD4+ T cells. In some embodiments, the encoded selectionmarker is ΔNGFR. The encoded selection marker may have the sequence of SEQ ID NO:3. The exogenous polynucleotide may comprise a sequence of SEQ ID NO:4. The encoded selection marker may be a truncated form of human EGFR polypeptide.

In some embodiments, the method further comprises the step of isolating the genetically-modified CD4⁺ T cells expressing the selection marker, thereby generating an enriched population of genetically-modified CD4^{IL-10} cells.

In some embodiments, at least 90% of the genetically-modified CD4⁻ T cells in the enriched population express a selection marker. In some embodiments, at least 95% of the genetically-modified CD4⁺ T cells in the enriched population express a selection marker. In some embodiments, at least 96, 97, 98, or 99% of the genetically-modified CD4⁺ T cells in the enriched population express a selection marker.

In some embodiments, at least 90% of the genetically-modified CD4⁺ T cells in the enriched population express IL-10. In some embodiments, at least 95% of the genetically-modified CD4⁺ T cells in the enriched population express IL-10. In some embodiments, at least 96, 97, 98, or 99% of the genetically-modified CD4⁺ T cells in the enriched population express IL-10.

In some embodiments, the method further comprises the step of incubating the enriched population of the genetically-modified CD4⁺ T cells. In some embodiments, the incubation is performed in the presence of anti-CD3 antibody and anti-CD28 antibody, or anti-CD3 antibody and anti-CD28 antibody coated beads. In some embodiments, the incubation is performed further in presence of IL-2. The incubation may be performed in the presence of feeder cells. In some embodiments, the incubation is performed in the presence of nanopreparations of anti-CD3 antibody and anti-CD28 antibody. The incubation may be performed in the presence of T Cell TransAct^{™} from Miltenyi Biotec. The incubation may be performed in the presence of ImmunoCult Human T Cell Activator^{™} from STEMCELL Technologies.

The method may further comprise the step of freezing the genetically-modified CD4⁺ T cells.

The primary CD4⁺ T cells may be from donors selected based on their HLA haplotypes. The method may further comprise the step of selecting T cell donors by analyzing their genetic information. The method may comprise the step of analyzing genetic information or HLA haplotype of potential T cell donors.

The primary CD4⁺ T cells may be from donors having at least a partial HLA match with a host to be treated with the primary CD4⁺ T cells or a modification thereof. The primary CD4⁺ T cells may be from donors having at least a partial HLA match with a stem cell (HSC), tissue or organ donor. The primary CD4⁻ T cells may be obtained from third party donors who are not biologically related with a host. The primary CD4⁺ T cells may be obtained from third party donors who are not biologically related with a stem cell, tissue or organ donor.

In step (i), the primary CD4⁺ T cells may have been obtained from three, four, five, six, seven, eight, nine, or ten different T cell donors. In some embodiments, the at least three T cell donors have at least 5/10, 6/10, 7/10, 8/10, or 9/10 match at the HLA-A, HLA-B, HLA-C, HLA-DRB1, and HLA-DQB1 loci to each other. In some embodiments, the at least three T cell donors have at least 4/8, 5/8, 6/8, 7/8, or 8/8 match at the HLA-A, HLA-B, HLA-C, and HLA-DRB1 loci to each other. In some embodiments, the at least three T cell donors have 2/2 match at the HLA-A locus to each other. In some embodiments, the at least three T cell donors have 2/2 match at the HLA-B locus to each other. In some embodiments, the at least three T cell donors have 2/2 match at the HLA-C locus to each other. In some embodiments, the at least three T cell donors have at least 3/4 or 4/4 match at the HLA-DRB1 and HLA-DQB1 loci to each other. In some embodiments, each of the at least three T cell donorshas an A*02 allele.

In some embodiments, in step (i), the primary CD4⁺T cells are obtained from one or more frozen stocks. In some embodiments, in step (i), the primary CD4⁺T cells are obtained from unfrozen peripheral blood mononuclear cells of the at least three different T cell donors. The method may further comprise the step of isolating CD4⁺ T cells from the peripheral blood mononuclear cells. In step (i), the primary CD4⁺T cells may be obtained from a liquid suspension. The liquid suspension may be obtained from a previously frozen stock.

CD4⁺ T cells from donors may be contacted with patient antigen-presenting cells (monocytes, dendritic cells, or DC-10 cells), generating allo-specificCD4⁺ T cells that are then modified to produce high levels of IL-10 (allo-CD4^{IL-10} cell).

In some instances, the method does not comprise the step of anergizing the CD4⁺ T cells in the presence of peripheral blood mononuclear cells (PBMCs) from a host. In some instances, the method does not comprise the step of anergizing the CD4⁺ T cells in the presence of recombinant IL-10 protein, wherein the recombinant IL-10 protein is not expressed from the CD4⁺ T cells. In some instances, the method does not comprise the step of anergizing the CD4⁺ T cells in the presence of DC10 cells from a host.

### 6.7. Methods of using poly-donor CD4^{IL-10} cells

In yet another aspect, the present disclosure provides the poly-donor CD4^{IL-10} cells of the invention or the pharmaceutical composition described herein for use in a method of treating a patient in need of immune tolerization, comprising the step of administering the poly-donor CD4^{IL-10} cells or the pharmaceutical composition to the patient.

The method may further comprise the preceding step of thawing a frozen suspension of poly-donor CD4^{IL-10} cells.

The poly-donor CD4^{IL-10} cells or the pharmaceuticalcomposition may prevent or reduce severity of pathogenic T cell response in the patient.

The treatment method may further comprise monitoring poly- donor CD4^{IL-10} cells in a patient after administration. The method may comprise the step of detecting a selection marker in a biological sample obtained from the patient, thereby detecting presence or absence of poly-donor CD4^{IL-10} T cells. The selection marker may be detected at multiple time points to trace changes in presence of poly-donor CD4^{IL-10} cells in a patient. The biological sample may be a biopsy or blood sample from the patient.

The poly-donor CD4^{IL-10} T cells are administered in a therapeutically effective amount. The amount can be determined based on the body weight and other clinical factors. 10³ to 10⁹ cells/kg may be administered. 10³ to 10⁸ cells/kg may be administered. 10³ to 10⁷ cells/kg may be administered. 10³ to 10⁶ cells/kg may be administered. 10³ to 10⁵ cells/kg may be administered. 10³ to 10⁴ cells/kg may be administered.

Poly-donor CD4^{IL-10} T cells may be administered on a therapeutically effective schedule. Poly-donor CD4^{IL-10} T cells may be administered once. Poly-donor CD4^{IL-10} cells may be administered every day,every 3 days, every 7 days, every 14 days, every 21 days, or every month.

The poly-donor CD4^{IL-10} T cells can be administered according to different administration routes, such as systemically, subcutaneously, or intraperitoneally. The cells may be administered within a saline or physiological solution which maycontain 2-20%, preferably 5 % human serum albumin.

### 6.7.1. Methods of reducing or preventing GvHD

The poly-donor CD4^{IL-10} cells or the pharmaceutical composition comprising poly-donor CD4^{IL-10} cells may be for use in a method of treating a patient before a hematopoietic stem cell (HSC) transplant (HSCT), concurrently with an HSCT, or following an HSCT.

The HSCT may be a matched related HSCT. The HSCT may be a haploidentical HSCT, a mismatched related HSCT, or a mismatched unrelated HSCT.

The patient may have a hematological malignancy which requires treatment with allo-HSCT. The hematological malignancy may be mediated by aberrant myeloid cells.

T cell donors may be selected based on genetic information of a patient to be treated with poly-donor CD4^{IL-10} cells and HSC, and/or genetic information of the HSC donor. T cell donors may be selected based on HLA haplotype of a patient to be treated with poly-donor CD4^{IL-10} cells and HSC, and/or HLA haplotype of the HSCdonor. The method may further comprise the step, prior to administering CD4^{IL-10} cells, of analyzing genetic information or HLA haplotype of T cell donors. The method may further comprise the step of analyzing genetic information or HLAhaplotype of a host. The method may further comprise the step of analyzing genetic information or HLA haplotype of an HSC donor.

In some instances, T cell donors, a host and an HSC donor are not biologically related. T cell donors, a host and an HSC donor may have different HLA haplotypes. T cell donors, a host and an HSC donor may have at least partial mismatch in HLA haplotype. T cell donors may be selected when they have HLA haplotype with an HLA match over a threshold value.

The HSC donor may be partially HLA mismatched to the patient. The HSC donor may have less than 5/10, 6/10, 7/10, 8/10, 9/10 or 10/10 match at the HLA-A, HLA-B, HLA-C, HLA-DRB1, and HLA-DQB1 loci to the patient. The HSC donor may have less than 4/8, 5/8, 6/8, 7/8, or 8/8 match at the HLA-A, HLA- B, HLA-C, and HLA-DRB1 loci to the patient. The HSC donor may have less than 2/2 match at the HLA-A, HLA-B, or HLA-C locus to the patient. The HSC donor may have less than 3/4 or 4/4 match at the HLA-DRB1 and HLA-DQB1 loci to the patient.

One or more of the T cell donors may be HLA-mismatched or partially HLA-mismatched to the patient. One or more of the T cell donors may have less than 5/10, 6/10, 7/10, 8/10, 9/10 or 10/10 match at the HLA-A, HLA-B, HLA- C, HLA-DRB1, and HLA-DQB1 loci to the patient. One or more of the Tcell donors may have less than 4/8, 5/8, 6/8, 7/8, or 8/8 match at the HLA-A, HLA-B, HLA-C, and HLA-DRB1 loci to the patient. One or more of the T cell donors may have less than 2/2 match at the HLA-A, HLA-B, or HLA-C locus to the patient. One or more of the T cell donors may have less than 2/4, 3/4 or 4/4 match at the HLA-DRB1 and HLA-DQB1 loci to the patient.

One or more of the T cell donors may be HLA-mismatched or partially HLA-mismatched with the HSC donor. One or more of the T cell donors may have less than 5/10, 6/10, 7/10, 8/10, 9/10 or 10/10 match at the HLA-A, HLA-B, HLA- C, HLA-DRB1, and HLA-DQB1 loci to the HSC donor. One or more of the T cell donors may have less than 4/8, 5/8, 6/8, 7/8, or 8/8 match at the HLA-A, HLA-B, HLA-C, and HLA-DRB1 loci to the HSC donor. One or more of the T cell donors may have less than 2/2 match at the HLA-A, HLA-B, or HLA-C locus to the HSC donor. One or more of the T cell donors may have less than 3/4 or 4/4 match at the HLA- DRB1 and HLA-DQB1 loci to the HSC donor. In some embodiments, the poly-donor CD4^{IL-10}cells or the pharmaceutical composition prevents or reduces severity of GvHD by the transplanted hematopoietic stem cells.

The poly-donor CD4^{IL-10} cells or the pharmaceutical composition may prevent or reduce severity of pathological T cell response by the transplanted hematopoietic cells. In specific embodiments, the poly-donor CD4^{IL-10} cells prevents or reduces GvHD.

### 6.7.2. Methods of treating cancer

Poly-donor CD4^{IL-10} cells may be used for treatment of cancer. The poly-donor CD4^{IL-10} cells may directly mediate anti-tumor effects (Graft versus Tumor, GvT), and an anti-leukemic effect (Graft versusLeukemia, GvL).

Poly-donor CD4^{IL-10} cells may be administered in combination with allogeneic mononuclear cells or PBMC for treatment of cancer. Poly-donor CD4^{IL-10} cells may be administered prior to or subsequence to administration of PBMC. Poly-donor CD4^{IL-10} cells and allogeneic mononuclear cells or PBMC may be administered concurrently.

Poly-donor CD4^{IL-10} cells and allogeneic mononuclear cells or PBMC may be administered at 1:3, 1:2, 1:1, 2:1 or 3:1 ratio.

The neoplastic cells may express CD13. The neoplastic cells may express HLA-class I. The neoplastic cells may express CD54. The neoplastic cells may express CD13, HLA-class I and CD54. The neoplastic cells may express CD112. The neoplastic cells may express CD58. The neoplastic cells may express CD155. The tumor may express CD112, CD58, or CD155. The tumor may be a solid or hematological tumor.

The patient may have a cancer selected from the group consisting of: Adrenal Cancer, Anal Cancer, Bile Duct Cancer, Bladder Cancer, Bone Cancer, Brain/CNS Tumors In Adults, Brain/CNS Tumors In Children, Breast Cancer, Breast Cancer In Men, Cancer of Unknown Primary, Castleman Disease, Cervical Cancer, Colon/Rectum Cancer, Endometrial Cancer, Esophagus Cancer, Ewing Family Of Tumors, Eye Cancer, Gallbladder Cancer, Gastrointestinal Carcinoid Tumors, Gastrointestinal Stromal Tumor (GIST), Gestational Trophoblastic Disease, Hodgkin Disease, Kaposi Sarcoma, Kidney Cancer, Laryngeal and Hypopharyngeal Cancer, Leukemia, Acute Lymphocytic (ALL), Acute Myeloid (AML, including myeloid sarcoma and leukemia cutis), Chronic Lymphocytic (CLL), Chronic Myeloid (CML) Leukemia, Chronic Myelomonocytic (CMML), Leukemia in Children, Liver Cancer, Lung Cancer, Lung Cancer with Non-Small Cell, Lung Cancer with Small Cell, Lung Carcinoid Tumor, Lymphoma, Lymphoma of the Skin, Malignant Mesothelioma, Multiple Myeloma, Myelodysplastic Syndrome, Nasal Cavity and Paranasal Sinus Cancer, Nasopharyngeal Cancer, Neuroblastoma, Non-Hodgkin Lymphoma, Non-Hodgkin Lymphoma In Children, Oral Cavity and Oropharyngeal Cancer, Osteosarcoma, Ovarian Cancer, Pancreatic Cancer, Penile Cancer, Pituitary Tumors, Prostate Cancer, Retinoblastoma, Rhabdomyosarcoma, Salivary Gland Cancer, Sarcoma - Adult Soft Tissue Cancer, Skin Cancer, Skin Cancer - Basal and Squamous Cell, Skin Cancer - Melanoma, Skin Cancer - Merkel Cell, Small Intestine Cancer, Stomach Cancer, Testicular Cancer, Thymus Cancer, Thyroid Cancer, Uterine Sarcoma, Vaginal Cancer, Vulvar Cancer, Waldenstrom Macroglobulinemia, and Wilms Tumor.

The cancer may be a myeloid tumor. The cancer may be AML or CML.

The method may be used to treat a hematological cancer affecting blood, bone marrow, and lymph nodes. The hematologicalcancer may be a lymphoma (e.g. Hodgkin's Lymphoma), lymphocytic leukemias, myeloma.
The hematological cancer may be acute or chronic myelogenous (myeloid)leukemia (AML, CML), or a myclodysplastic syndrome.

The cancer may be refractory or resistant to a therapeutic intervention.

The poly-donor CD4^{IL-10} cells may be used in combination with a therapeutic intervention. The combination may be simultaneous or performed at different times. Preferably the therapeutic intervention is selected from the group consisting of: chemotherapy, radiotherapy, allo-HSCT, immune suppression, blood transfusion, bone marrow transplant, growth factors, biologicals.

The poly-donor CD4^{IL-10} cells may induce cell death of tumor infiltrating myeloid lineage cells (e.g., monocytes, macrophages, neutrophils).

### 6.7.3. Methods of treating other disorders

In some embodiments, poly-donor CD4^{IL-10} cells are administered to treat autoimmune disease.

In some embodiments, the autoimmune disease is selected from the group consisting of: type-1 diabetes, autoimmune uveitis, rheumatoid arthritis, psoriasis, psoriatic arthritis, multiple sclerosis, systemic lupus, inflammatory bowel disease, Addison's disease, Graves' disease, Sjögren's syndrome, Hashimoto's thyroiditis, myasthenia gravis, autoimmune vasculitis, pernicious anemia, ulcerative colitis, bullous diseases, scleroderma, and celiac disease. The autoimmune disease may be Crohn's disease, ulcerative colitis, celiac disease, type-1 diabetes, lupus, psoriasis, psoriatic arthritis, or rheumatoid arthritis. In some embodiments, the patient has an allergic or atopic disease. The allergic or atopic disease can be selected from the group consisting of: asthma, atopic dermatitis, and rhinitis. The patient may have a food allergy.

Poly-donor CD4^{IL-10} cells may be administered to prevent or reduce severity of pathogenic T cell response to cell and organ transplantation other than HSCT. In some embodiments, the method comprises the step of organ transplantation to the patient, either prior to or subsequent to administration of poly-donor CD4^{IL-10} T cells or the pharmaceutical composition. The organ may be a kidney, a heart, or pancreatic islet cells. In preferred embodiments, the poly-donor CD4^{IL-10} cells or the pharmaceutical composition prevents or reduces severity of host rejection of the organ transplantation.

Poly-donor CD4^{IL-10} cells may be administered to prevent or reduce immune response associated with gene therapy, e.g., administration of recombinant AAV (rAAV). The method may further comprise the step of administering a recombinant AAV to the patient, either prior to or subsequent to administration of the poly-donorCD4^{IL-10} cells or the pharmaceutical composition.

Poly-donor CD4^{IL-10} cells may be administered to prevent or reduce immune response associated with transplantation of iPS-derived tissues or cells. The iPS-derived tissues and cells include, but are not limited to cardiomyocytes, hepatocytes, epithelialcells, cartilage, bone and muscle cells, neurons.

Poly-donor CD^{4IL-10} cells may be administered to treat inflammation. The inflammation can be related to coronary artery disease (CAD), Type 2 diabetes, neurodegenerative diseases, or inflammatory bowel disease, but is not limited thereto.

In some embodiments, poly-donor CD4^{IL-10} cells are administered to treat a disease or disorder involving hyperactivity of NLPR3 inflammasome. Poly-donor CD4^{IL-10} cells may be administered to treat a disease or disorder involving increased IL-1β production by activated monocytes, macrophages or dendritic cells. Poly-donor CD4^{IL-10} cells may be adm inistered to treat a disease or disorder involving increased IL-18 production by activated monocytes, macrophages or dendritic cells. Poly-donor CD^{4IL-10} cells may be administered to treat a disease or disorder involving increased mature caspase 1 production by activated monocytes, macrophages or dendritic cells.

Poly-donor CD^{4IL-10} cells may be administered to reduce IL-1β production by activated monocytes, macrophages or dendritic cells. Poly-donor CD^{4IL-10} cells may be administered to reduce IL-18 production by activated monocytes, macrophages or dendritic cells. Poly-donor CD^{4IL-10} cells may be administered to reduce mature caspase 1 production by activated monocytes, macrophages or dendritic cells.

Poly-donor CD4^{IL-10} cells may be administered to reduce patient hyperactive immune response to viral infection. The virus may be SARS-coV-2. Poly-donor CD4^{IL-10} cells may be administered to reduce hyperactive immune responses to bacterial infections, such as toxic shock and cytokine storm.

### 6.8. Examples

The following examples are provided by way of illustration not limitation.

### 6.8.1. Example 1: Generation of poly-donor CD4^{IL-10} cells

### Vector production

Poly-donor CD4^{IL-10} cells were produced by transduction with a lentiviral vector containing coding sequences of both the human IL-10 and a truncated form of the NGFR (ΔNGFR) (FIGs. 1 and 2), as described in WO2016/146542. The sequence of the vector is provided as SEQ ID NO:5. In short, the lentiviralvector was generating by ligating the coding sequence of human IL-10 from 549 bp fragment of pH15C (ATCC 68192)) into plasmid #1074.1071.hPGK.GFP.WPRE.mhCMV.dNGFR.SV40PA.

The presence of the bidirectional promoter (human PGK promoter plus minimal core element of the CMV promoter in the opposite direction) allows co-expression of the two transgenes. The plasmid further contains a coding sequence of an antibiotic resistance gene (e.g., ampicillin or kanamycin).

The lentiviral vectors were produced by Ca₃PO₄ transient four-plasmid co-transfection into 293T cells and concentrated by ultracentrifugation: 1 µM sodium butyrate was added to the cultures for vector collection. Titer was estimated on 293T cells by limiting dilution, and vector particles were measured by HIV-1 Gag p24 antigen immune capture (NEN Life Science Products; Waltham, MA). Vector infectivity was calculated as the ratio between titer and particle. For concentrated vectors, titers ranged from 5×10⁸ to 6×10⁹ transducing units/ml, and infectivity from 5×10⁴ to 5×10⁵ transducing units/ng.

### Production of CD4^{IL-10} cells

FIG. 3 is a schematic representation of the production process of CD4^{IL-10} cells. CD4⁺ T cells from healthy donors were purified. Human CD4⁺ T cells were activated with soluble anti-CD3, soluble anti-CD28 mAbs, and rhIL-2 (50 U/mL) for 48 hours before transduction with a bidirectional lentiviral vector encoding for human IL-10 and a truncated form the human NGF receptor (LV-IL-10/ΔNGFR) at multiplicity of infection (MOI) of 20.

After 11 days, transduced cells were analyzed by FACS for the expression of ΔNGFR, and the vector copy number (VCN) was quantified by digital droplet PCR (ddPCR).

The mean transduction efficiency of CD4⁺ T cells from 10 different donors was 45 ± 17% with VCN of 2.7 ± 0.6%. FIG. 4A shows percentages of CD4⁺ΔNGFR⁺ cells (mean± SD, n=10 left bar) and vector copy numbers (VCN, mean ± SD, n=10 right bar) in human CD4⁺ T cells transduced with LV-IL-10/ΔNGFR (a bidirectional lentiviral vector encoding for human IL-10 and a truncated form the human NGF receptor). The frequency of CD4⁺ΔNGFR⁺ cells and the vector copy numbers were quantified by digital droplet PCR (ddPCR) in CD4^{IL-10} cells.

ΔNGFR' T cells were purified using anti-CD271 mAb-coated microbeads and resulted in > 95% pure CD4^{IL-10} cells populations. After purification, cells were stained with markers for CD4 and ΔNGFR and analyzed by FACS. The data showed purity resulting from the purification step was over 98%. FIG. 4B shows FACS data from two representative donors (Donor B and Donor C) out of 10 donors tested. The purified CD4^{IL-10} cells were restimulated 3 times at 14 day intervals and their *in vitro* and *in vivo* functions were tested after the second (TF2) and or third restimulation (TF3) functions.

Resting CD4^{IL-10} cells produced IL-10 constitutively. Upon activation, the level of IL-10 produced was strongly enhanced.

### CD4^{IL-10} cells have a cytokine production profile which is comparable to that of naturally derived Tr1 cells.

Cytokine production profiles of single donor CD4^{IL-10} cells were analyzed after the second (TF2) and third (TF3) restimulation and the results are provided in FIG. 5. Specifically, CD4^{IL-10} cells (2×10⁵ cells in 200 µl) were restimulated as previously described (Andolfi et al. Mol Ther. 2012;20(9):1778-1790 and Locafaro et al.Mol Ther. 2017;25(10):2254-2269). At day 14, after the 2^{nd} round (TF2) and 3^{rd} round (TF3) of restimulation, CD4^{IL-10} cells were left unstimulated (orange bar) or stimulated with immobilized anti-CD3 and soluble anti-CD28 mAbs (grey bars) for 48hrs. Culture supernatants were collected and levels of IL-10, IL-4, IL-5, IFN-γ and IL-22 were determined by ELISA. All samples were tested in triplicate. Mean± SD, n=8 donors tested are presented. The results provided in FIG. 5 show that CD4^{IL-10} cells stimulated with immobilized anti-CD3 and soluble anti-CD28 mAbs show a Tr1 cell cytokine production profile.

Although considerable variations between the different donors were observed, the overall cytokine production profiles after the second (TF2) or the third (TF3) restimulation were comparable and reflected those of Tr1 cells (Roncarolo et al., Immunity, 2018). Like Tr1 cells, the CD4^{IL-10} cells produced high levels of IL-10, IL-5, IFN-γ and IL-22. but low levels of IL-4 and undetectable levels of IL-2.

### CD4^{IL-10} cells express high levels of Granzyme B and selectively kill myeloid leukemia cells

The CD4^{IL-10} cells were further analyzed after the 2^{nd} round (TF2) of restimulation for expression of granzyme B (GzB). The data in FIG. 6A show that most of the CD4^{IL-10} cells expressed GzB. More than 95 % of all CD4^{IL-10} cells derived from 7 different donors expressed high levels of Granzyme B.

The CD4^{IL-10} cells from the 2^{nd} round (TF2) of restimulation were further analyzed for their cytotoxic effects against myeloid leukemia cells (ALL-CM) and an erythroid leukemia cell line (K562). CD4^{IL-10} cells (10⁵/well) were co-cultured with K562 and ALL-CM cells (10⁵/well) at 1:1 ratio for 3 days. Residual leukemic cell lines (CD45^{low}CD33⁺) were counted by FACS for each target cell.

The CD4^{IL-10} cells selectively killed the myeloid leukemia cells (ALL-CM) as shown in FIG. 6B. The % of killed ALL-CM cells varied between 62% and 100%, whereas the killing of the erythroid leukemia cell line K562 (which are highly sensitive for nonspecific cytotoxic activities) varied between 0 and 27% (4 different donors tested). Taken together, these data confirm that CD4^{IL-10} cells express Granzyme B and efficiently kill myeloid leukemia cells. As expected, some variations in the killing capacity of the CD4^{IL-10} cells from individual donors was observed.

### CD4^{IL-10} cells suppress the proliferative responses of both allogeneic CD4⁺ and CD8⁺ T cells

The CD4^{IL-10} cells were also analyzed for their effects on allogeneic CD4⁺ T cells or CD8⁺ T cells. Specifically, allogeneic PBMC cells were labeled with eFluor^{®} 670 (10⁵ cells/well) and stimulated with allogeneic mature dendritic (DC) cells (5×10⁴ cells/well) and soluble anti-CD3 mAbs in the absence or presence of CD4^{IL-10} cells (10⁵ cells/well) at a 1:1 Responder: Suppressor ratio. After 4 days of culture, the percentages of proliferating responder cells were determined by eFluor^{®} 670 dilution with flow cytometry after gating on CD4⁺ΔNGFR⁻ T cells or CD8⁺ΔNGFR⁻ T cells. FIGs. 7A and 7B show effects of CD4^{IL-10} cells from six different, unpooled, donors (Donor-C, Donor-E, and Donor-F in FIG. 7A and Donor-H, Donor-I, and Donor-L in FIG. 7B) on CD4⁺ T cells with percentages of proliferation and suppression FIGs. 8A and 8B show effects of CD4^{IL-10} cells from six different, unpooled, donors (Donor -C, Donor-E, and Donor-F in FIG. 8A and Donor-H, Donor-I, and Donor-L in FIG. 8B) on CD8⁺ T cells.

The results demonstrated that CD4^{IL-10} cells from 6 different donors, unpooled and tested separately, downregulated the proliferative responses of both allogeneic CD4⁺ and CD8' T cells. The suppressive effects on the CD4' T-cells varied between 51% and 96%, while the suppressive effects on the CD8⁺ T-cells varied between 62% and 73 %.

### Production and characterization of poly-donor CD4^{IL-10} cells

CD4^{IL-10} cells were generated as described above and FIG. 3 using CD4⁺ cells from multiple donors. CD4^{IL-10} cells from each donor were stimulated by the second (TF2) and third (TF3) restimulation. After the third stimulation, CD4^{IL-10} cells from the three donors were pooled at a 1:1:1 ratio and stimulated with immobilized anti-CD3 and soluble anti-CD28 mAbs for 48hrs.

### Poly-donor CD4^{IL-10} cells have a cytokine production profile which is comparable to that of CD4^{Il-10} cells of individual donors and Tr1 cells.

Culture supernatants were collected and levels of IL-10, IL-4, IL-5, IFN-γ and IL-22 were determined by ELISA. The results provided in FIG. 9 show that the cytokine production of polydonor CD4^{IL-10} cells pooled from 3 different allogeneic donors (pooled 1:1:1) (red dot) was comparable to that of CD4^{IL-10} cells from individual donor (n=8) derived CD4^{IL-10} cells (gray bars). The poly-donor CD4^{IL-10} cells produced high levels of IL-10, IL-5, IFN-γ and IL-22 and low levels of IL-4 and undetectable levels of IL-2 (not shown). These data indicate that it is feasible to pool CD4^{IL-10} cells and that these poly-donor CD4^{IL-10} cells maintain the cytokine production signature of single donor derived CD4^{IL-10} cells and Tr1 cells. Importantly, the pooled allogeneic cell populations contained > 95% viable cells indicating that they did not kill each other.

### Poly-donor CD4^{IL-10} cells express high levels of Granzyme B and kill myeloid leukemia cell lines.

The poly-donor CD4^{IL-10} cells were further analyzed after 3^{rd} round (TF3) of restimulation for expression of granzyme B (GzB). The data in FIG. 10A show that most of the poly-donor CD4^{IL-10} cells express GzB. Over 95 % of the polydonor CD4^{IL-10} cells expressed Granzyme B, comparable to the GzB expression of single donor derived CD4^{IL-10} cells.

The CD4^{IL-10} cells from 3^{rd} round (TF3) of restimulation were further analyzed for their cytotoxic effects on myeloid leukemia cells (ALL-CM cell line) or K562. The poly-donor CD4^{IL-10} cells (10⁵/well) were co-cultured with K562 and ALL-CM cells (10⁵/well) at 1:1 ratio for 3 days. Residual leukemic cell lines (CD45^{low}CD33⁺) were counted by FACS for each target cell. The results provided in FIG. 10B show that some level of cytotoxicity against K562 cells, which are highly sensitive for nonspecific cytotoxicity. Nevertheless, a level of selectivity towards myeloid leukemia cells (ALL-CM) was obtained which is comparable to that of single donor derived CD4^{IL-10} cells.

### Poly-donor CD4^{IL-10} cells suppress the proliferative responses of both allogeneic CD4+ and CD8+ T cells.

The poly-donor CD4^{IL-10} cells were also analyzed for their effects on allogeneic CD4⁺ T cells or CD8⁺ T cells. Specifically, allogeneic PBMC cells were labeled with eFluor^{®} 670 (10⁵ cells/well) and stimulated with allogenic mature dendritic (DC) cells (5×10⁴ cells/well) and soluble anti-CD3 mAbs in the absence or presence of poly-donor CD4^{IL-10} cells (10⁵ cells/well) at a 1:1 Responder: Suppressor ratio. After 4 days of culture, the percentages of proliferating responder cells were determined by eFluor^{®} 670 dilution with flow cytometry after gating on CD4⁺ΔNGFR⁻ T cells and CD8⁺ΔNGFR⁻ T cells. FIG. 11A shows results from poly-donor CD4^{IL-10} cells containing CD4^{IL-10} cells from Donor-C, Donor-E, and Donor-F. FIG. 11B shows results from poly-donor CD4^{IL-10} cells containing CD4^{IL-10} cells from Donor-H, Donor-I, and Donor-L, which had been frozen, stored and thawed prior to testing.

FIG. 11A shows that the poly-donor CD4^{IL-10} cells (from 3 different donors) suppress CD4⁺ and CD8⁺ T-cell responses by 96% and 74%, respectively. Comparable results were obtained with a second, different batch of poly-donor CD4^{IL-10} cells which was tested after the cells had been frozen, stored and thawed prior to testing (FIG. 11B). Suppression of CD4⁺ and CD8⁺ T cell proliferation was 68% and 75 %, respectively. These data indicate that poly-donor CD4^{IL-10} cells can be frozen and stored without loss of function.

Collectively the data obtained with poly-donor CD4^{IL10} cells indicate that these cell preparations can be pooled without any problems. They contain > 95 % viable cells and maintain all the relevant functions (cytokine production, cytotoxic capacity, and suppression of allogeneic T cell responses) of single donor CD4^{IL-10} cells. The use of larger pools of poly-donor CD4^{IL-10} cells should reduce the natural variations observed between CD4^{IL-10} cell lots originating from different individual donors, and should provide a large quantity of off-the-shelf CD4^{IL-10} cells for human therapy.

A poly-donor CD4^{IL-10} cell product will have significant advantages in terms of a more homogeneous product which will allow the determination of well defined, less lot-to-lot variation, potency, and release criteria. In addition, it will enable the development of a continuous large-scale cell production process.

### Other methods for production of poly-donor CD4^{IL-10} cells

Before the lentiviral transduction, buffy coats from minimally 3-5 different donors are pooled. CD4⁺ cells are isolated from buffy coats by positive selection using anti-CD4 antibody. Purity of the pooled CD4⁺ cells is checked by FACS. Alternatively, frozen human CD4^{I} cells are obtained from minimally 3-5 normal healthy donors. The frozen human CD4^{I} cells are thawed before use. CD4⁺ cells from buffy coats or frozen stocks are activated for 24-48 hrs by a combination of CD3 and CD28 antibodies or CD3- and CD28 antibody coated beads in the presence of IL-2. In some cases, CD4⁺ cells from buffy coats or frozen stocks are activated with soluble anti-CD3, soluble anti-CD28 mAbs, and rhIL-2 (50 U/mL) for 48 hours and transduced with a bidirectional lentiviral vector encoding for human IL-10 as described above for production of CD4^{IL-10} cells.

In some cases, the HLA haplotype of the T cell donors (or CD4⁺ cells isolated from the donors) are first determined and CD4⁺ cells having desired HLA haplotypes are selectively pooled and used.

Poly-donor CD4^{IL-10} cells are generated by transducing the activated CD4⁺ cells described above with the lentiviral vector containing human IL-10 and ΔNGFR coding sequences described above.

On Day 7-11, which is 5-9 days after the transduction, the cells are harvested and successfully transduced T cells purified utilizing an anti-NGFR antibody. This process generally results in 95% pure populations of poly-donor CD4^{IL-10} cells.

The purified poly-donor CD4^{IL-10} cells are counted and re-stimulated by a mixture of CD3- and CD28 antibodies, CD3- and CD28 antibody coated beads, optionally in the presence of feeder cells for another 8-10 days in the presence of IL-2. In some cases, the purified poly-donor CD4^{IL-10} cells are re-stimulated in the presence of feeder cells.

After a total culture period of 14-18 days, CD4^{IL-10} cells are harvested, counted and tested for their capacity to produce IL-10 spontaneously or following activation with CD3 and CD28 antibodies or CD3 and CD28 antibody coated beads. Additionally, the levels of GrzB and perforin are measured. Their capacity to suppress human T cell (PBMC) and purified CD4⁺ and CD8⁺ T cell proliferation are also tested.

In addition, the production of IL-22 is measured both constitutively and following activation of 200,000 CD4^{IL-10} cells in a volume of 200 microliter using a combination of CD3 and CD28 antibodies as described previously for the production of other cytokines such as IFNγ, IL-10, IL-4 and IL-5. IL-22 production levels are measured in IL-22 specific ELISA as described for the other cytokines in WO2016/146542. The pooled CD4^{IL-10} cells are frozen before storage.

### 6.8.2. Example 2:Treatment or prevention of GvHD using poly-donor CD4^{IL-10} cells

### Effects of poly-donor CD4^{IL-10} cells in vivo.

A population of poly-donor CD4^{IL-10} cells were tested in a humanized xeno GvHD disease model, an NSG mouse model, for their effect on GvHD induced by human PBMC as illustrated in FIG. 12. NSG mice were sub-lethally irradiated and intravenously injected with human PBMC (5×10⁶ cells/mouse), with poly-donor (three donors) CD4^{IL-10} cells (5×10⁶ cells/mouse), or with human PBMC (5×10⁶ cells/mouse) in combination with poly-donor CD4^{IL-10} cells (three donors) (5x10⁶ cells/mouse). GvHD was evaluated as previously described (Bondanza et al. Blood 2006) based on weight loss (>20% weight loss), skin lesions, fur condition, activity, and hunch.

FIG. 13 shows % of NSG mice demonstrating GvHD on each day after injection. Administration of 5x 10⁶ human PBMC to irradiated NSG mice resulted unexpectedly in an unusually fulminant GvHD. All mice died at day 10 which reflects very lethal GvHD. Co-adminstration of 5x10⁶ poly-donor CD4^{IL-10} cells delayed this fulminant GvHD, but the mice were sacrificed at day 14 because they reached the prespecified humane 20 % body weight loss criterion for sacrifice (Fig 13). Nevertheless, these results indicate that poly-donor CD4^{IL-10} can delay very severe GvHD. Importantly, poly-donor CD4^{IL-10} cells administered alone at the same dose as the PBMC (5×10⁶ cells ) failed to induce any sign of GvHD.

The presence of human CD4^{IL-10} cells were also tested in the spleen (FIG. 14, left panels) and bone marrow (FIG. 14, right panels) of the NSG mice injected with human PBMC (5×10⁶ cells/mouse), poly-donor (three donors) CD4^{IL-10} cells (5×10⁶ cells/mouse), or human PBMC (5×10⁶ cells/mouse) in combination with poly-donor CD4^{IL-10} cells (three donors) (5×10⁶ cells/mouse) at 14 days post injection. The results provided in FIG. 14 show that poly-donor CD4^{IL-10} cells migrated to spleen and bone marrow. Low percentages of these cells were found to be present 14 days after infusion of the cells. These results indicate that poly-donor CD4^{IL-10} cells delayed fulminant GvHD induced by human PBMC and that they do not themselves induce any xeno GvHD.

### Poly-donor CD4^{IL-10} cells inhibit severe xeno GvHD by purified CD4⁺ cells.

Poly-donor CD4^{IL-10} cells were tested in a humanized xeno GvHD model in which GvHD disease was induced by administration of 2.5 X 10⁶ purified human CD4+ T cells as illustrated in FIG. 15. NSG mice were sub-lethally irradiated at day 0 and on day 3 were intravenously injected with human CD4⁺ T cells (2.5x10⁶ cells/mouse) alone or in combination with poly-donor CD4^{IL-10} cells (three different donors) (2.5x10⁶ cells/mouse) or with CD4^{IL-10} cells from a single donor from the pool (2.5x10⁶ cells/mouse). GvHD was evaluated as previously described (Bondanza et al. Blood 2006) based on weight loss (>20% weight loss), skin lesions, fur condition, activity, and hunch.

FIG. 16 shows % of NSG mice demonstrating GvHD on each day after injection. The results show that poly-donor CD4^{IL-10} cells can inhibit GvHD mediated by human allogeneic CD4⁺ T cells. In this particular experiment, xeno GvHD was very severe, because all mice in the control group which received CD4+ T cells were dead at day 20. In contrast, co-administration of 2.5 x 10⁶ poly-donor CD4^{IL-10} inhibited GvHD by 75 %. Single-donor CD4^{IL-10} cells were also protective but the effects were less potent.

### Other experiments

Therapeutic effects of the poly-donor CD4^{IL-10} cells are tested in four different groups of mice: (i) mice receiving human PBMC from a donor unrelated to the CD4^{IL-10} cells (GvHD positive control); (ii) mice receiving the poly-donor CD4^{IL-10} cells (negative control); (iii) mice receiving a combination of PBMC and the poly-donor CD4^{IL-10} cells at 1:1 ratio; and (iv) mice receiving a combination of PBMC and the poly-donor CD4^{IL-10} cells at 2:1 ratio or at different ratios. Among animals receiving combination of PBMC and the poly-donor CD4^{IL-10} cells, some animals receive PBMC and the poly-donor CD4^{IL-10} cells concurrently, some animals receive poly-donor CD4^{IL-10} cells several days (e.g., 5 days) after receiving PBMC, and some animals receive poly-donor CD4^{IL-10} cells several days (e.g., 5 days) before receiving PBMC.

The mice are monitored for development of GvHD by measuring weight at weeks 1, 2, 3, 4, and if necessary week 5, after administration of PBMC and/or the poly-donor CD4^{IL-10} cells. In addition to weight loss, the mice will be inspected for skin lesions, fur condition and activity. The mice in the treatment groups are monitored for additional periods to determine effects of the poly-donor CD4^{IL-10} cells on long term survival.

The amount and localization of the poly-donor CD4^{IL-10} cells are also monitored in peripheral blood and tissues after administration. Specifically, presence of poly-donor CD4^{IL-10} cells are monitored in peripheral blood and at sites of inflammation: lymph nodes, spleen, gut, and bone marrow. The mice in the treatment group(s) are monitored for an additional 3 weeks to determine long-term survival.

The results demonstrate that poly-donor CD4^{IL-10} cells are effective in reducing and preventing xeno-GvHD.

### 6.8.3. Example 3: Inhibition of GvHD and treatment of cancer

A population of poly-donor CD4^{IL-10} cells are tested in an NSG mouse model transplanted with human PBMC and AML tumor cells for their effect on xeno-GvHD induced by human PBMC and anti-tumor effects. AML cells (ALL-CM) are administered i.v. as described previously in WO 2016/146542. PBMC or poly-donor CD4^{IL-10} cells or combinations thereof are administered 3 days later.

Poly-donor CD4^{IL-10} cells are obtained as described in Example 1. Therapeutic effects of the poly-donor CD4^{IL-10} cells are tested in four different groups of mice, each having received irradiation and 5x10⁶ ALL-CM cells (AML mice) at day 0: (i) AML mice without additional treatment; (ii) AML mice receiving 5x10⁶ human PBMC from a donor unrelated to the poly-donor CD4^{IL-10} cells - the PBMCs cause severe xeno-GvHD; (iii) AML mice receiving 2.5x10⁶ poly-donor CD4^{IL-10} cells; and (iv) AML mice receiving combinations of PBMC and the poly-donor CD4^{IL-10} cells at 1:1 or 2:1 ratio or at different ratios. One additional group of mice do not receive ALL-CML cells but receive 5x10⁶ human PBMC at day 3 after irradiation.

Effects of the poly-donor CD4^{IL-10} cells on xeno-GvHD induced by human PBMC are tested based on weight loss, skin lesions, fur condition, activity, death rate and long-term survival. Anti-tumor or graft versus leukemia (GvL) effects of the poly-donor CD4^{IL-10} cells are tested based on reduction of tumor cells in the circulation and long-term tumor free survival.

Some mice are monitored for up to 7 weeks in order to monitor long-term survival and complete tumor remissions.

Results demonstrate that poly-donor CD4^{IL-10} cells are effective in both inhibition of xeno-GvHD and treatment of cancer.

### 6.8.4. Example 4: Treatment of cancer using poly-donor CD4^{IL-10} cells

A population of poly-donor CD4^{IL-10} cells are tested in an ALL-CM leukemia model of T cell therapy in NSG mice.

NSG mice were sub-lethally irradiated and intravenously injected with myeloid leukemia cells (ALL-CM) (5x10⁶) at day 0. In the first group of animals, PBMC (5x10⁶) or single donor (from donor BC-I and donor BC-H) CD4^{IL-10} cells (2.5x10⁶) were injected at day 3. In the second group of animals, PBMC (5x10⁶) or poly-donor CD4^{IL-10} cells (2.5x10⁶) were injected at day 3. Graft-versus-leukemia (GvL) effect was tested in the animals based on reduction of circulating leukemia cells and long-term leukemia free survival. Leukemia was measured as previously described (Locafaro G. et al Molecular Therapy 2017).

As provided in FIG. 17A and FIG. 17B, all of the mice injected with ALL-CM myeloid leukemia cells had extensive leukemia progression at day 17. Administration of 5x10⁶ PBMC resulted in a strong inhibition of leukemia progression. Interestingly, a comparable level of inhibition of leukemia progression was obtained by lower number (2.5x10⁶) of single-donor CD4^{IL10} (Fig17A) or poly-donor CD4^{IL10} (Fig17B). These data indicate that single donor and poly-donor CD4^{IL10} have strong direct anti leukemia effects.

Graft-versus-leukemia (GvL) effects of single-donor CD4^{IL10} and poly-donor CD4^{IL10} were further tested in combination with PBMC in mice injected with ALL-CM myeloid leukemia cells. Administration of 5x10⁶ PBMC resulted in a strong inhibition of leukemia progression and administration of 5x10⁶ PBMC combined with single donors CD4^{IL10} (2.5x10⁶) had synergistic effect (Fig18A). Interestingly, administration of 5x10⁶ PBMC combined with 2.5x10⁶ poly-donor CD4^{IL10} had a comparable synergistic GvL effect (Fig18B). These data indicate that poly-donor CD4^{IL10} act in synergy with PBMC to mediate strong GvL effects.

### 6.8.5. Example 5: Treatment of chronic inflammatory and autoimmune diseases using poly-donor CD4^{IL-10} cells

Activation of the NLPR3 inflammasome has been implicated in many chronic inflammatory and autoimmune diseases. The NLPR3 inflammasome can be activated by "danger signals" which lead to caspase1-mediated production of the pro-inflammatory cytokines IL-1β and IL-18 by monocytes/macrophages. A series of in vitro experiments are performed to investigate the effects of poly-donor CD4^{IL-10} cells on the NLPR3 inflammasome and IL-1 β/IL-18 production by human monocytes.

First, human PBMC are isolated from peripheral blood by standard density centrifugation on Ficoll/Paque (Sigma-Aldrich). Monocytes are isolated from the human PBMC by negative selection using monocyte isolation kit II (Miltenyi) according to the manufacturer's instructions. Negative selection is preferred because positive selection or adherence can lead to undesired activation of the cells. Isolated monocytes are plated at 5x10⁴ cells/200 µl in the presence of 2x10⁵ or 1x10⁵ poly-donor CD4^{IL-10} cells /200 µl per well in 96-well microtiter plates in culture medium containing 3% toxin free human AB serum.

Table 1 summarizes treatment conditions applied to 17 sets of monocytes, each set including 6 wells of cells. It is known that LPS alone can activate human monocytes without a second signal provided by ATP.

| **Table 1** | | | | | |
|---|---|---|---|---|---|
| **Group** # | **Monocytes** | **CD4 cells/supernatant** | **YVADfmk*** | **LPS**** | **Other** |
| **Medium control** | | | | | |
| Incubation time: 1.5 hour; followed by activation by LPS for 4 hours | | | | | |
| 1 | Monocytes | No | No | No | |
| 2 | Monocytes | No | No | LPS | |
| 3 | Monocytes | No | YVADfmk | LPS | |

| **Co cultivation of monocytes and poly-donor CD4^{IL-10} cells** | | | | | |
|---|---|---|---|---|---|
| 4 | Monocytes | CD4^{IL-10} cells | No | No | |
| 5 | Monocytes | CD4^{IL-10} cells | No | LPS | |
| 6 | Monocytes | CD4^{IL-10} cells | YVADfmk | LPS | |

| **Co cultivation of monocytes and control CD4 GFP+ cells** | | | | | |
|---|---|---|---|---|---|
| 7 | Monocytes | CD4 GFP cells | No | No | |
| 8 | Monocytes | CD4 GFP cells | No | LPS | |
| 9 | Monocytes | CD4 GFP cells | YVADfmk | LPS | |

| **Cultivation of monocytes in the presence of supernatants*** of poly-donor CD4^{IL-10} cell or CD4 GFP+ cell culture** | | | | | |
|---|---|---|---|---|---|
| 10 | Monocytes | 50% supernatant of CD4^{IL-10} cells | No | LPS | |
| 11 | Monocytes | 25% supernatant of CD4^{IL-10} cells | No | LPS | |
| 12 | Monocytes | 12.5% supernatant of CD4^{IL-10} cells | No | LPS | |
| 13 | Monocytes | 50% supernatant of CD4^{IL-10} cells | No | LPS | Anti-IL-10 antibody |
| 14 | Monocytes | 50% supernatant of CD4 GFP cells | No | LPS | |
| 15 | Monocytes | 25% supernatant of CD4 GFP cells | No | LPS | |
| 16 | Monocytes | 12.5% supernatant of CD4 GFP cells | No | LPS | |
| 17 | Monocytes | 50% supernatant of CD4 GFP cells | No | LPS | Anti-IL-10 antibody |

| | | | | | |
|---|---|---|---|---|---|
| * YVADfmk is an inhibitor specific to caspase 1. 20 mM Z-YVADfmk (Biovision, Enzo Life Sciences, or Axxora Life Sciences) dissolved in DMSO is used. ** LPS (Signa-Aldrich) 100ng/ml *** Supernatants of CD4^{IL-10} and CD4 GFP or NGFR cultures are obtained by incubating CD4^{IL-10} or CD4 GFP cells at 1X10⁶/ml for 3days and collecting the supernatants. IL-10 production levels are measured by IL-10 specific ELISA. | | | | | |

After treatments outlined in Table 1, supernatants are collected from 6 wells for each group and IL-1 β/IL-18 production is measured by ELISA specific for mature IL-1β or IL-18 (Biolegend). Cells collected from 6 wells for Group #3, 10, 13, 14, and 17 are analyzed by Western Blot to determine levels of activated caspase 1.

Data from the experiments show that poly-donor CD4^{IL-10} cells down-regulate IL-1B and IL-18 production by activated monocytes. They further show that poly-donor CD4^{IL-10} cells down-regulate mature caspase-1 production in activated monocytes. Additionally, poly-donor CD4^{IL-10} and IL-10 produced by the poly-donor CD4^{IL-10} down-regulate inflammasome.

Similar experiments are performed with human macrophages or dendritic cells instead of monocytes. Results from the experiments demonstrate that poly-donor CD4^{IL-10} cells further down-regulate IL-1β, IL-18, and mature caspase-1 production from activated macrophages and dendritic cells.

These suggest that poly-donor CD4^{IL-10} cells can be used to treat diseases or disorders involving hyperactivation of NLPR3 inflammasome. In particular, poly-donor CD4^{IL-10} cells can be used to treat chronic inflammatory and autoimmune diseases. The NLPR3 inflammasome can be activated by exogenous or endogenous "danger signals", such as Pathogen Associated Molecular Patterns (PAMPs), silica, asbestos, Danger Associated Molecular Patterns (DAMPs) like products from damaged mitochondria, necrotic and stressed cells, and uremic acid crystals.

### 6.8.6. Experimental methods and materials

**Cell preparation and cell lines.** Peripheral blood mononuclear cells (PBMC) were prepared by centrifugation over Ficoll-Hypaque gradients. CD4⁺ T cells were purified with a CD4 T cell isolation kit (Miltenyi Biotec, Bergisch Gladbach, Germany) with a resulting purity of >95%. Mature dendritic cells (DC) were generated from peripheral blood CD14⁺ monocytes positively selected using CD14⁺ MicroBeads (Miltenyi Biotech, Germany) according to the manufacturer's instructions and cultured in RPM1 1640 (Lonza, Italy) supplemented with 10% fetal bovine serum (FBS; Lonza, Italy), 100 U/mL penicillin/streptomycin (Lonza, Italy), 2 mM L-glutamine (Lonza, Italy), at 37°C in the presence of 10 ng/mL recombinant human (rh) IL-4 (R&D Systems, Minneapolis MN, USA) and 100 ng/mL rhGM-CSF (Genzyme, Seattle, WA, USA) for 5 days and matured with 1 mg/mL of lipopolysaccharide (LPS, Sigma, CA, USA) for an additional two days.

**Plasmid construction.** The coding sequence of human IL-10 was excised from pH15C (ATCC n° 68192), and the 549bp fragment was cloned into the multiple cloning site of pBluKSM (Invitrogen) to obtain pBIuKSM-hIL-10. A fragment of 555bp was obtained by excision of hIL-10 from pBluKSM-hIL-10 and ligation to 1074.1071.hPGK.GFP.WPRE.mhCMV.dNGFR.SV40PA (here named LV-ΔNGFR), to obtain LV-IL-10/ΔNGFR. The presence of the bidirectional promoter (human PGK promoter plus minimal core element of the CMV promoter in opposite direction) allows co-expression of the two transgenes (Locafaro et al. Mol Ther. 2017;25(10):2254-2269). The sequence of LV-IL-10/ΔNGFR was verified by pyrosequencing (Primm).

**Vector production and titration.** VSV-G-pseudotyped third generation bidirectional lentiviral vectors were produced by Ca₃PO₄ transient four-plasmid co-transfection into 293T cells and concentrated by ultracentrifugation as described (Locafaro et al. Mol Ther. 2017;25(10):2254-2269). Titer was estimated by limiting dilution, vector particles were measured by HIV-1 Gag p24 antigen immune capture (NEN Life Science Products; Waltham, MA), and vector infectivity was calculated as the ratio between titer and particle. Titers ranged from 5x10⁸ to 6x10⁹ transducing units/mL, and infectivity from 5x10⁴ to 10⁵ transducing units/ng of p24.

**Generation of CD4^{IL-10} cell lines.** Polyclonal CD4-transduced cells were obtained as previously described (Andolfi et al. Mol Ther. 2012;20(9):1778-1790). Briefly, CD4 purified T cells were activated for 48 hours with soluble anti-CD3 monoclonal antibody (mAb, 30 ng/mL, OKT3, Janssen-Cilag, Raritan, NJ, USA), anti-CD28 mAb (1 µg/mL, BD) and rhIL-2 (50 U/mL, PROLEUKIN, Novartis, Italy). T cells were transduced with LV-IL-10/ΔNGFR (CD4^{IL-10}) with multiplicity of infection (MOI) of 20. At day 11, CD4⁺ΔNGFR⁺ cells were beads-sorted using CD271⁺ Microbeads (Miltenyi Biotec, Bergisch Gladbach, Germany) and expanded in X-VIVO15 medium with 5% human serum (BioWhittaker-Lonza, Washington), 100 U/mL penicillin-streptomycin (BioWhittaker), and 50 U/mL rhIL-2 (PROLEUKIN, Novartis, Italy). At day 7 and 10, medium was replaced by fresh medium supplemented with 50U/mL of rhIL-2. At day 14, cells were collected, washed, and restimulated with allogeneic feeder mixture as previously described (Andolfi et al. Mol Ther. 2012;20(9):1778-1790). After 14 days, cells were collected and frozen. Thawed CD4^{IL-10} cells were restimulated and after the 2^{nd} and 3^{rd} re-stimulation and expansion were functionally characterized *in vitro* and used for *in vivo* experiments.

**Vector Copy Number Analysis.** Cells were cultured for at 11 days after transduction in order to get rid of non-integrated vector forms. Genomic DNA was isolated with QIAamp DNA Blood Mini Kit (QIAGEN, 51106), according to the manufacturer's instructions. Vector integrations were quantified by QX200 Droplet Digital PCR System (Bio-Rad), according to the manufacturer's instructions.

**Cytokine determination.** To measure cytokine production, after 2^{nd} and 3^{rd} re-stimulation single donor and poly-donor CD4^{IL-10} cells were left unstimulated or stimulated with immobilized anti-CD3 (10 µg/mL) and soluble anti-CD28 (1µg/mL) mAbs in a final volume of 200 µl of medium (96 well round-bottom plates, 2x10⁵/well). Supernatants were harvested after 48 hours of culture and levels of IL-10, IL-4, IL-5, IFN-γ and IL-22 were determined by ELISA according to the manufacturer's instructions (BD Biosciences).

**Flow cytometry analysis.** For the expression of Granzyme B (clone MHGB04, Invitrogen, USA) after surface staining with CD4, CD4^{IL-10} cells were fixed, permeabilized, and stained using the BD Cytofix/Cytoperm^{™} Kit according to the manufacturer's instructions (Cat. No. 554714, Biolegend, USA). Stained cells were washed two times with PBS supplemented with 1% FBS and analysed with a BD LSRFortessa analysed utilizing FlowJo 10 software.

**Killing assays.** After 2^{nd} and 3^{rd} re-stimulation, cytotoxicity of single-donor and poly-donor CD4^{IL-10} cells was analysed in co-culture experiments. Briefly, non-mycloid leukemia and a myeloid leukemia cell lines, K562 and ALL-CM respectively, were used as target cells and plated with CD4^{IL-10} cells at 1:1 ratio (10⁵ target cells and 10⁵ CD4^{IL-10} cells) for 3 days. At the end of co-culture, cells were harvested and K562 and ALL-CM cells were analysed and counted by FACS.

**Suppression assays.** To measure the suppressive capacity of single donor and poly-donor CD4^{IL-10} cells, allogeneic PBMC were labeled with Cell Proliferation Dye eFluor^{®} 670 (Invitrogen, CA, USA), according to manufacturer's instructions prior to stimulation with allogeneic mature DC (5x10⁴ cells/well) and soluble anti-CD3 (50 ng/mL) mAb. PBMC and suppressor cells were added at a 1:1 ratio (10⁵ PBMC and 10⁵ CD4^{IL-10} cells). After 3 days of culture, proliferation of responder cells was determined by analyzing the eFluor670 dilution of CD4⁺ΔNGFR⁻ or CD8⁺ΔNGFR⁻ T cells by FACS.

**Graft-versus Host Disease models:** In all experiments 6/8 week-old female NSG mice were used. On day 0 mice received total body irradiation with a single dose of 175-200 cGy from a linear accelerator according to the weight of the mice, and were intravenously with PBMC cells (5x10⁶), or CD4^{IL-10} cells (single-donors or poly-donor - pool of three donors - 5x10⁶ or 2.5x10⁶), or with PBMC (5x10⁶) in combination with CD4^{IL-10} cells (5×10⁶ or 2.5x10⁶). Survival, weight loss, activity, fur, skin, and hunch were monitored at least 3 times per week as previously described (Bondanza et al. Blood. 2006;107(5):1828-1836). Mice were euthanized for ethical reasons when their loss of bodyweight was 20%.

Alternatively, on day 0 mice received total body irradiation as above. On day 3 mice were injected with CD4⁺ T cells (2.5x10⁶), single and poly-donor (pool of three donors) CD4^{IL-10} cells (2.5x10⁶), or CD4⁺ T cells (2.5x10⁶) in combination with single and poly-donor (pool of three donors) CD4^{IL-10} cells (2.5x10⁶). GvHD induction was monitored as indicated above.

### 9. SEQUENCES

SEQ ID NO: 1 (Human IL-10 amino acid sequence) 
M H S S A L L C C L V L L T G V R A S P G Q G T Q S E N S C T H F P
G N L P N M L R D L R D A F S R V K T F F Q M K D Q L D N L L L K
E S L L E D F K G Y L G C Q A L S E M I Q F Y L E E V M P Q A E N Q
D P D I K A H V N S L G E N L K T L R L R L R R C H R F L P C E N
K S K A V E Q V K N A F N K L Q E K G I Y K A M S E F D I F I N Y I
E A Y M T M K I R N
SEQ ID NO: 2 (Human IL-10 exemplary nt sequence) 
ggggtgagggccagcccaggccagggcacccagtctgagaacagctgcacccacttccca
ggcaacctgcctaacatgcttcgagatctccgagatgccttcagcagagtgaagactttc
tttcaaatgaaggatcagctggacaacttgttgttaaaggagtccttgctggaggacttt
aagggttacctgggttgccaagccttgtctgagatgatccagttttacctggaggaggtg
atgccccaagctgagaaccaagacccagacatcaaggcgcatgtgaactccctgggggag
aacctgaagaccctcaggctgaggctacggcgctgtcatcgatttcttccctgtgaaaac
aagagcaaggccgtggagcaggtgaagaatgcctttaataagctccaagagaaaggcatc
tacaaagccatgagtgagtttgacatcttcatcaactacatagaagcctacatgacaatgaagatacgaaactgagtc
SEQ ID NO: 3 (ΔNGFR amino acid sequence) 
L E D P P V P P L E G Y V G H K A H N H S S Q D G A I D R D E V V
G G A S G H H G L G A A H H C G H H T C H R A G Y E V L F W R C
L R L L G A G G C A V R A L W G C G P C N P T A R D L L A L G V
G P A C A L A E L A L G V L A H G A G Q A R V H V V G L V G I R A
V G A L L A H G V L L V L A G E H E A R A R L A H A A R L A A P
S R L I L V V A V G A A A H G V V G L H A R R R H A L E P H A L G
A R L A R L G R A H H V G E R H A V Q A G L T H G L V G S T R L
G H T L A Q V A G F A A L T A V C V Q A C G A C L L G T S K G H P
Q K Q Q Q Q A R P V H G A A G G T C P H
SEQ ID NO: 4 (ΔNGFR exemplary nt sequence) 
ctagaggatccccctgttccacctcttgaaggctatgtaggccacaaggcccacaaccac
agcagccaggatggagcaatagacagggatgaggttgtcggtggtgcctcgggtcaccac
gggctgggagctgcccatcactgtggtcaccacacctgccaccgtgctggctatgaggtc
ttgttctggaggtgcctcaggctcctgggtgctgggggctgtgctgtccgagccctctgg
gggtgtggaccgtgtaatccaacggccagggatctcctcgcactcggcgtcggcccagcg
tgtgcactcgcggagctggcgctcggtgtcctcgcacacggtgcagggcaggcacgggtc
cacgtggttggcctcgtcggaatacgtgccgtcggggcactcctcgcacacggtgttctg
cttgtcctggcaggagaacacgaggcccgagcccgcctcgcacacgcggcacgcctcgca
gcgcccagtcgtctcatcctggtagtagccgtaggcgcagcggcacacggcgtcgtcggc
ctccacgcacggcgccgacatgctctggagccccacgcactcggtgcacggcttgcacgg
ctcggtcgcgctcaccacgtcggagaacgtcacgctgtccaggcagggctcacacacggt
ctggttggctccacaaggctgggccacaccctcgcccaggttgcaggctttgcagcactc
accgctgtgtgtgtacaggcctgtggggcatgcctccttggcacctccaagggacacccc
cagaagcagcaacagcagcaggcgcggcccgtccatggcgcggccggtggcacctgcccc cat
SEQ ID NO: 5 (nucleotide sequence of bd.ΔNGFR.PGK.IL-10)

aaatttcacaaataaagcatttttttcactgcattctagttgtggtttgtccaaactcatcaatgtatcttatcatgtctggctctagctatcccgcccc
taactccgcccagttccgcccattctccgccccatggctgactaattttttttatttatgcagaggccgaggccgcctcggcctctgagctattc
cagaagtagtgaggaggcttttttggaggcctaggcttttgcgtcgagacgtacccaattcgccctatagtgagtcgtattacgcgcgctcact
ggccgtcgttttacaacgtcgtgactgggaaaaccctggcgttacccaacttaatcgccttgcagcacatccccctttcgccagctggcgtaa
tagcgaagaggcccgcaccgatcgcccttcccaacagttgcgcagcctgaatggcgaatggcgcgacgcgccctgtagcggcgcattaa
gcgcggcgggtgtggtggttacgcgcagcgtgaccgctacacttgccagcgccctagcgcccgctcctttcgctttcttcccttcctttctcg
ccacgttcgccggctttccccgtcaagctctaaatcgggggctccctttagggttccgatttagtgctttacggcacctcgaccccaaaaaact
tgattagggtgatggttcacgtagtgggccatcgccctgatagacggtttttcgccctttgacgttggagtccacgttctttaatagtggactctt
gttccaaactggaacaacactcaaccctatctcggtctattcttttgatttataagggattttgccgatttcggcctattggttaaaaaatgagctg
atttaacaaaaatttaacgcgaattttaacaaaatattaacgtttacaatttcccaggtggcacttttcggggaaatgtgcgcggaacccctattt
gtttatttttctaaatacattcaaatatgtatccgctcatgagacaataaccctgataaatgcttcaataatattgaaaaaggaagagtatgagtatt
caacatttccgtgtcgcccttattcccttttttgcggcattttgccttcctgtttttgctcacccagaaacgctggtgaaagtaaaagatgctgaag
atcagttgggtgcacgagtgggttacatcgaactggatctcaacagcggtaagatccttgagagttttcgccccgaagaacgttttccaatga
tgagcacttttaaagttctgctatgtggcgcggtattatcccgtattgacgccgggcaagagcaactcggtcgccgcatacactattctcagaa
tgacttggttgagtactcaccagtcacagaaaagcatcttacggatggcatgacagtaagagaattatgcagtgctgccataaccatgagtga
taacactgcggccaacttacttctgacaacgatcggaggaccgaaggagctaaccgcttttttgcacaacatgggggatcatgtaactcgcc
ttgatcgttgggaaccggagctgaatgaagccataccaaacgacgagcgtgacaccacgatgcctgtagcaatggcaacaacgttgcgca
aactattaactggcgaactacttactctagcttcccggcaacaattaatagactggatggaggcggataaagttgcaggaccacttctgcgct
cggcccttccggctggctggtttattgctgataaatctggagccggtgagcgtgggtctcgcggtatcattgcagcactggggccagatggt
aagccctcccgtatcgtagttatctacacgacggggagtcaggcaactatggatgaacgaaatagacagatcgctgagataggtgcctcac
tgattaagcattggtaactgtcagaccaagtttactcatatatactttagattgatttaaaacttcatttttaatttaaaaggatctaggtgaagatcct
ttttgataatctcatgaccaaaatcccttaacgtgagttttcgttccactgagcgtcagaccccgtagaaaagatcaaaggatcttcttgagatcc
tttttttctgcgcgtaatctgctgcttgcaaacaaaaaaaccaccgctaccagcggtggtttgtttgccggatcaagagctaccaactctttttcc
gaaggtaactggcttcagcagagcgcagataccaaatactgtccttctagtgtagccgtagttaggccaccacttcaagaactctgtagcacc
gcctacatacctcgctctgctaatcctgttaccagtggctgctgccagtggcgataagtcgtgtcttaccgggttggactcaagacgatagtta

ccggataaggcgcagcggtcgggctgaacggggggttcgtgcacacagcccagcttggagcgaacgacctacaccgaactgagatacc
tacagcgtgagctatgagaaagcgccacgcttcccgaagggagaaaggcggacaggtatccggtaagcggcagggtcggaacaggag
agcgcacgagggagcttccagggggaaacgcctggtatctttatagtcctgtcgggtttcgccacctctgacttgagcgtcgatttttgtgatg
ctcgtcaggggggcggagcctatggaaaaacgccagcaacgcggcctttttacggttcctggccttttgctggccttttgctcacatgttcttt
cctgcgttatcccctgattctgtggataaccgtattaccgcctttgagtgagctgataccgctcgccgcagccgaacgaccgagcgcagcga
gtcagtgagcgaggaagcggaagagcgcccaatacgcaaaccgcctctccccgcgcgttggccgattcattaatgcagctggcacgaca
ggtttcccgactggaaagcgggcagtgagcgcaacgcaattaatgtgagttagctcactcattaggcaccccaggctttacactttatgcttc
cggctcgtatgttgtgtggaattgtgagcggataacaatttcacacaggaaacagctatgaccatgattacgccaagcgcgcaattaaccctc
actaaagggaacaaaagctggagctgcaagcttggccattgcatacgttgtatccatatcataatatgtacatttatattggctcatgtccaacat
taccgccatgttgacattgattattgactagttattaatagtaatcaattacggggtcattagttcatagcccatatatggagttccgcgttacataa
cttacggtaaatggcccgcctggctgaccgcccaacgacccccgcccattgacgtcaataatgacgtatgttcccatagtaacgccaatagg
gactttccattgacgtcaatgggtggagtatttacggtaaactgcccacttggcagtacatcaagtgtatcatatgccaagtacgccccctattg
acgtcaatgacggtaaatggcccgcctggcattatgcccagtacatgaccttatgggactttcctacttggcagtacatctacgtattagtcatc
gctattaccatggtgatgcggttttggcagtacatcaatgggcgtggatagcggtttgactcacggggatttccaagtctccaccccattgacg
tcaatgggagtttgttttggcaccaaaatcaacgggactttccaaaatgtcgtaacaactccgccccattgacgcaaatgggcggtaggcgtg
tacggtgggaggtctatataagcagagctcgtttagtgaaccggggtctctctggttagaccagatctgagcctgggagctctctggctaact
agggaacccactgcttaagcctcaataaagcttgccttgagtgcttcaagtagtgtgtgcccgtctgttgtgtgactctggtaactagagatcc
ctcagacccttttagtcagtgtggaaaatctctagcagtggcgcccgaacagggacctgaaagcgaaagggaaaccagagctctctcgac
gcaggactcggcttgctgaagcgcgcacggcaagaggcgaggggcggcgactggtgagtacgccaaaaattttgactagcggaggcta
gaaggagagagatgggtgcgagagcgtcagtattaagcgggggagaattagatcgcgatgggaaaaaattcggttaaggccaggggga
aagaaaaaatataaattaaaacatatagtatgggcaagcagggagctagaacgattcgcagttaatcctggcctgttagaaacatcagaagg
ctgtagacaaatactgggacagctacaaccatcccttcagacaggatcagaagaacttagatcattatataatacagtagcaaccctctattgt
gtgcatcaaaggatagagataaaagacaccaaggaagctttagacaagatagaggaagagcaaaacaaaagtaagaccaccgcacagc
aagcggccgctgatcttcagacctggaggaggagatatgagggacaattggagaagtgaattatataaatataaagtagtaaaaattgaacc
attaggagtagcacccaccaaggcaaagagaagagtggtgcagagagaaaaaagagcagtgggaataggagctttgttccttgggttctt
gggagcagcaggaagcactatgggcgcagcctcaatgacgctgacggtacaggccagacaattattgtctggtatagtgcagcagcaga
acaatttgctgagggctattgaggcgcaacagcatctgttgcaactcacagtctggggcatcaagcagctccaggcaagaatcctggctgtg
gaaagatacctaaaggatcaacagctcctggggatttggggttgctctggaaaactcatttgcaccactgctgtgccttggaatgctagttgg
agtaataaatctctggaacagattggaatcacacgacctggatggagtgggacagagaaattaacaattacacaagcttaatacactccttaa
ttgaagaatcgcaaaaccagcaagaaaagaatgaacaagaattattggaattagataaatgggcaagtttgtggaattggtttaacataacaa
attggctgtggtatataaaattattcataatgatagtaggaggcttggtaggtttaagaatagtttttgctgtactttctatagtgaatagagttagg
cagggatattcaccattatcgtttcagacccacctcccaaccccgaggggacccgacaggcccgaaggaatagaagaagaaggtggaga
gagagacagagacagatccattcgattagtgaacggatctcgacggtatcggttaacttttaaaagaaaaggggggattggggggtacagt
gcaggggaaagaatagtagacataatagcaacagacatacaaactaaagaattacaaaaacaaattacaaaaattcaaaattttatcgatcac
gagactagcctcgagagatctgatcataatcagccataccacatttgtagaggttttacttgctttaaaaaacctcccacacctccccctgaacc
tgaaacataaaatgaatgcaattgttgttgttaacttgtttattgcagcttataatggttacaaataaggcaatagcatcacaaatttcacaaataa
ggcatttttttcactgcattctagttttggtttgtccaaactcatcaatgtatcttatcatgtctggatctcaaatccctcggaagctgcgcctgtcat
cgaattcctgcagcccggtgcatgactaagctagctcagttagcctcccccatctcccctagaggatccccctgttccacctcttgaaggctat
gtaggccacaaggcccacaaccacagcagccaggatggagcaatagacagggatgaggttgtcggtggtgcctcgggtcaccacgggc
tgggagctgcccatcactgtggtcaccacacctgccaccgtgctggctatgaggtcttgttctggaggtgcctcaggctcctgggtgctggg
ggctgtgctgtccgagccctctgggggtgtggaccgtgtaatccaacggccagggatctcctcgcactcggcgtcggcccagcgtgtgca
ctcgcggagctggcgctcggtgtcctcgcacacggtgcagggcaggcacgggtccacgtggttggcctcgtcggaatacgtgccgtcgg
ggcactcctcgcacacggtgttctgcttgtcctggcaggagaacacgaggcccgagcccgcctcgcacacgcggcacgcctcgcagcg
cccagtcgtctcatcctggtagtagccgtaggcgcagcggcacacggcgtcgtcggcctccacgcacggcgccgacatgctctggagcc
ccacgcactcggtgcacggcttgcacggctcggtcgcgctcaccacgtcggagaacgtcacgctgtccaggcagggctcacacacggtc
tggttggctccacaaggctgggccacaccctcgcccaggttgcaggctttgcagcactcaccgctgtgtgtgtacaggcctgtggggcatg
cctccttggcacctccaagggacacccccagaagcagcaacagcagcaggcgcggcccgtccatggcgcggccggtggcacctgccc
ccatcgcccgcctcccgcggcagcgctcgacttccagctcggtccgctttgcggactgatggggctgcgctgcgctgcgctccagcgccc
cccctgcccgccggagctggccgcggcccgaattccgcggaggctggatcggtcccggtgtcttctatggaggtcaaaacagcgtggat
ggcgtctccaggcgatctgacggttcactaaacgagctctgcttatataggcctcccaccgtacacgcctaccctcgagaagcttgatatcga

attcccacggggttggggttgcgccttttccaaggcagccctgggtttgcgcagggacgcggctgctctgggcgtggttccgggaaacgc
agcggcgccgaccctgggtctcgcacattcttcacgtccgttcgcagcgtcacccggatcttcgccgctacccttgtgggccccccggcga
cgcttcctgctccgcccctaagtcgggaaggttccttgcggttcgcggcgtgccggacgtgacaaacggaagccgcacgtctcactagtac
cctcgcagacggacagcgccagggagcaatggcagcgcgccgaccgcgatgggctgtggccaatagcggctgctcagcggggcgcg
ccgagagcagcggccgggaaggggcggtgcgggaggcggggtgtggggcggtagtgtgggccctgttcctgcccgcgcggtgttcc
gcattctgcaagcctccggagcgcacgtcggcagtcggctccctcgttgaccgaatcaccgacctctctccccagggggatccccggtctg
caggaattcatgcacagctcagcactgctctgttgcctggtcctcctgactggggtgagggccagcccaggccagggcacccagtctgag
aacagctgcacccacttcccaggcaacctgcctaacatgcttcgagatctccgagatgccttcagcagagtgaagactttctttcaaatgaag
gatcagctggacaacttgttgttaaaggagtccttgctggaggactttaagggttacctgggttgccaagccttgtctgagatgatccagtttta
cctggaggaggtgatgccccaagctgagaaccaagacccagacatcaaggcgcatgtgaactccctgggggagaacctgaagaccctc
aggctgaggctacggcgctgtcatcgatttcttccctgtgaaaacaagagcaaggccgtggagcaggtgaagaatgcctttaataagctcca
agagaaaggcatctacaaagccatgagtgagtttgacatcttcatcaactacatagaagcctacatgacaatgaagatacgaaactgagtcg
agaatcaacctctggattacaaaatttgtgaaagattgactggtattcttaactatgttgctccttttacgctatgtggatacgctgctttaatgcctt
tgtatcatgctattgcttcccgtatggctttcattttctcctccttgtataaatcctggttgctgtctctttatgaggagttgtggcccgttgtcaggca
acgtggcgtggtgtgcactgtgtttgctgacgcaacccccactggttggggcattgccaccacctgtcagctcctttccgggactttcgctttc
cccctccctattgccacggcggaactcatcgccgcctgccttgcccgctgctggacaggggctcggctgttgggcactgacaattccgtgg
tgttgtcggggaagctgacgtcctttccatggctgctcgcctgtgttgccacctggattctgcgcgggacgtccttctgctacgtcccttcggc
cctcaatccagcggaccttccttcccgcggcctgctgccggctctagagcctcttccgcgtcttcgccttcccgggtcgagctcggtaccttta
agaccaatgacttacaaggcagctgtagatcttagccactttttaaaagaaaaggggggactggaagggctaattcactcccaacgaagac
aagatctgctttttgcttgtactgggtctctctggttagaccagatctgagcctgggagctctctggctaactagggaacccactgcttaagcct
caataaagcttgccttgagtgcttcaagtagtgtgtgcccgtctgttgtgtgactctggtaactagagatccctcagacccttttagtcagtgtgg
aaaatctctagcagtagtagttcatgtcatcttattattcagtatttataacttgcaaagaaatgaatatcagagagtgagaggaacttgtttattgc
agcttataatggttacaaataaagcaatagcatcac

## Claims

1. A population of CD4⁺ T cells that have been genetically modified to comprise an exogenous polynucleotide encoding IL-10, wherein:
(a) the CD4⁺ T cells were obtained from at least three different T cell donors (poly-donor CD4^{IL-10} cells); and
(b) the exogenous polynucleotide is integrated into the T cell nuclear genome.

2. The population of CD4' T cells of claim 1, wherein the CD4' T cells in the population collectively have six, seven, eight, nine, ten, eleven, twelve, or more different HLA haplotypes.

3. The population of CD4⁺ T cells of claim 1, wherein:
(a) all the CD4⁺ T cells in the population have:
(i) at least 5/10, 6/10, 7/10, 8/10, or 9/10 match at the HLA-A, HLA-B, HLA-C, HLA-DRB1, and HLA-DQB1 loci to each other;
(ii) at least 4/8, 5/8, 6/8, 7/8, or 8/8 match at the HLA-A, HLA-B, HLA-C, and HLA-DRB1 loci to each other;
(iii) 2/2 match at the HLA-A locus to each other;
(iv) 2/2 match at the HLA-B locus to each other;
(v) 2/2 match at the HLA-C locus to each other; and/or
(vi) all the CD4⁺ T cells in the population have at least 3/4 or 4/4 match at the HLA-DRB1 and HLA-DQB1 loci with each other; and/or
(b) all the CD4⁺ T cells in the population have an A*02 allele.

4. The population of CD4⁺ T cells of claim 1 or claim 2, wherein the exogenous polynucleotide further comprises:
(a) a sequence encoding a selection marker, optionally wherein the selection marker is ΔNGFR; and/or
(b) lentiviral vector sequences.

5. The population of CD4⁺ T cells of any one of the above claims, wherein:
(a) at least 90% of the CD4⁺ T cells within the population express IL-10;
(b) the genetically modified CD4' T cells constitutively express at least 100pg, 200pg, 500pg, 1ng, 5ng, 10ng, or 50ng IL-10 per 10⁶ of the CD4⁻ T cells/ml of culture medium; and/or
(c) the genetically modified CD4⁺ T cells express at least 1ng, 2ng, 5ng, 10ng, 100ng, 200ng, or 500ng IL-10 per 10⁶ of the CD4⁺ T cells/ml after activation with anti-CD3 and anti-CD28 antibodies.

6. The population of CD4⁺ T cells of claim 4(a), wherein at least 90%, at least 95%, or at least 98% of the CD4⁺ T cells within the population express the selection marker from the exogenous polynucleotide.

7. The population of CD4⁺ T cells of any one of the above claims, wherein the genetically modified CD4⁺ T cells express CD49b, LAG-3, TGF-β, IFNγ, GzB, perforin, CD18, CD2, CD226, and/or IL-22.

8. The population of CD4⁺ T cells of any one of the above claims, wherein the CD4⁺ T cells are in:
(a) a frozen suspension; or
(b) a liquid suspension, optionally wherein the liquid suspension has previously been frozen.

9. A method of making poly-donor CD4^{IL-10} cells, comprising the steps of:
(i) modifying primary CD4⁺ T cells which have been obtained from at least three different T cell donors, wherein each donor's CD4⁺ T cells are separately modified by introducing an exogenous polynucleotide encoding IL-10, and
(ii) pooling the genetically modified CD4⁺ T cells,
thereby obtaining the poly-donor CD4^{IL-10} cells.

10. The method of claim 9, further comprising the step, before step (i), after step (i), after step (i) and before step (ii), or after step (ii), of:
incubating the primary CD4⁺ T cells in the presence of:
(a) an anti-CD3 antibody, and anti-CD28 antibody; or
(b) anti-CD3 antibody and anti-CD28 antibody coated beads,
optionally wherein the method further comprises incubating the primary CD4⁺ T cells in the presence of IL-2.

11. The method of claim 9 or claim 10, wherein the exogenous polynucleotide further comprises a segment encoding a selection marker, optionally wherein the encoded selection marker is ΔNGFR;
optionally wherein the method further comprises the step, after step (i), of:
isolating the genetically-modified CD4⁺ T cells expressing the selection marker, thereby generating an enriched population of genetically-modified CD4⁺ T cells.

12. The method of any one of claims 9-11, wherein in step (i), the primary CD4⁺ T cells are obtained from one or more frozen stocks.

13. The population of CD4' cells according to any one of claims 1-8 for use in a method of treating a patient in need of immune tolerization, wherein the method comprises the step of:
administering the poly-donor CD4^{IL-10} cells to the patient.

14. The population of CD4⁺ cells for use according to claim 13, wherein the method further comprises the step of:
administering hematopoietic stem cells (HSC) of an HSC donor to the patient either prior to or subsequent to administration of the pooled donor CD4^{IL-10} cells.

15. The population of CD4⁺ cells for use according to claim 13, wherein the patient has:
(a) an inflammatory or autoimmune disease, optionally wherein the inflammatory or autoimmune disease is selected from the group consisting of type-1 diabetes, Crohn's disease, autoimmune uveitis, rheumatoid arthritis, psoriasis, psoriatic arthritis, multiple sclerosis, systemic lupus, inflammatory bowel disease, Addison's disease, Graves' disease, Sjögren's syndrome, Hashimoto's thyroiditis, myasthenia gravis, autoimmune vasculitis, pernicious anemia, ulcerative colitis, bullous diseases, scleroderma, and celiac disease;
(b) a disease or disorder involving hyperactivity of NLPR3 inflammasome;
(c) type 2 diabetes, a neurodegenerative disease, a cardiovascular disease or inflammatory bowel disease; and/or
(d) an allergic or atopic disease, optionally wherein the allergic or atopic disease is selected from the group consisting of: asthma, atopic dermatitis, and rhinitis.

16. The population of CD4⁺ cells for use according to claim 13, wherein the method further comprises the step of organ transplantation to the patient, either prior to or subsequent to administration of the population of CD4⁺ T cells.

17. The population of CD4⁺ cells according to any one of claims 1-8 for use in a method of treating a hematological cancer, the method comprising:
administering to a hematological cancer patient an amount of poly-donor CD4^{IL-10} cells sufficient to induce anti-cancer effect,
wherein the poly-donor CD4^{IL-10} cells are genetically modified by vector-mediated gene transfer of the coding sequence of human IL-10 under control of a constitutive promoter.

18. The population of CD4⁺ cells for use according to claim 17, wherein:
(a) the poly-donor CD4^{IL-10} cells are administered to the patient prior to or subsequent to administration of an allogenic hematopoietic stem cell transplant (allo HSCT), and the amount of poly-donor CD4^{IL-10} cells administered to the patient is sufficient to suppress GvHD without suppressing graft versus leukemia (GvL) or graft versus tumor (GvT) efficacy of the allo HSCT; and/or
(b) the hematological cancer is a myeloid leukemia.

19. The population of CD4⁺ cells for use according to claim 13, wherein the patient has Crohn's disease.

## Patentansprüche

1. Eine Population von CD4⁺-T-Zellen, die genetisch modifiziert wurden, um ein exogenes Polynucleotid zu umfassen, das für IL-10 kodiert, wobei:
(a) die CD4⁺-T-Zellen aus mindestens drei verschiedenen T-Zell-Spendern (Polydonor-CD4^{IL-10}-Zellen) erhalten wurden; und
(b) das exogene Polynucleotid in das T-Zellkerngenom integriert ist.

2. Population von CD4⁺-T-Zellen nach Anspruch 1, wobei die CD4⁺-T-Zellen in der Population kollektiv sechs, sieben, acht, neun, zehn, elf, zwölf oder mehr verschiedene HLA-Haplotypen aufweisen.

3. Population von CD4⁺-T-Zellen nach Anspruch 1, wobei:
(a) alle CD4⁺-T-Zellen in der Population Folgendes aufweisen:
(i) eine Übereinstimmung von mindestens 5/10, 6/10, 7/10, 8/10 oder 9/10 an den HLA-A-, HLA-B-, HLA-C-, HLA-DRB1- und HLA-DQB1-Genorten miteinander;
(ii) eine Übereinstimmung von mindestens 4/8, 5/8, 6/8, 7/8 oder 8/8 an den HLA-A-, HLA-B-, HLA-C- und HLA-DRB1-Genorten miteinander;
(iii) eine 2/2-Übereinstimmung an dem HLA-A-Genort miteinander;
(iv) eine 2/2-Übereinstimmung an dem HLA-B-Genort miteinander;
(v) eine 2/2 -Übereinstimmung an dem HLA-C-Genort miteinander; und/oder
(vi) alle CD4⁺-T-Zellen in der Population eine Übereinstimmung von mindestens 3/4 oder 4/4 an den HLA-DRB1- und HLA-DQB1-Genorten miteinander aufweisen; und/oder
(b) alle CD4⁺-T-Zellen in der Population ein A*02-Allel aufweisen.

4. Population von CD4⁺-T-Zellen nach Anspruch 1 oder Anspruch 2, wobei das exogene Polynucleotid ferner Folgendes umfasst:
(a) eine Sequenz, die für einen Selektionsmarker kodiert, wobei der Selektionsmarker optional ΔNGFR ist; und/oder
(b) Lentivirusvektorsequenzen.

5. Population von CD4⁺-T-Zellen nach einem der vorhergehenden Ansprüche, wobei:
(a) mindestens 90% der CD4⁺-T-Zellen innerhalb der Population IL-10 exprimieren;
(b) die genetisch modifizierten CD4⁺-T-Zellen konstitutiv mindestens 100 pg, 200 pg, 500 pg, 1 ng, 5 ng, 10 ng oder 50 ng IL-10 pro 10⁶ CD4⁺-T-Zellen/ml Kulturmedium exprimieren; und/oder
(c) die genetisch modifizierten CD4⁺-T-Zellen mindestens 1 ng, 2 ng, 5 ng, 10 ng, 100 ng, 200 ng oder 500 ng IL-10 pro 10⁶ CD4⁺-T-Zellen/ml nach Aktivierung mit Anti-CD3- und Anti-CD28-Antikörpern exprimieren.

6. Population von CD4⁺-T-Zellen nach Anspruch 4(a), wobei mindestens 90%, mindestens 95% oder mindestens 98% der CD4⁺-T-Zellen innerhalb der Population den Selektionsmarker von dem exogenen Polynucleotid exprimieren.

7. Population von CD4⁺-T-Zellen nach einem der vorhergehenden Ansprüche, wobei die genetisch modifizierten CD4⁺-T-Zellen CD49b, LAG-3, TGF-β, IFNγ, GzB, Perforin, CD18, CD2, CD226 und/oder IL-22 exprimieren.

8. Population von CD4⁺-T-Zellen nach einem der vorhergehenden Ansprüche, wobei die CD4⁺-T-Zellen in Folgendem sind:
(a) einer gefrorenen Suspension; oder
(b) einer flüssigen Suspension, wobei die flüssige Suspension optional zuvor gefroren war.

9. Ein Verfahren zur Herstellung von Polydonor-CD4^{IL-10}-Zellen, das die folgenden Schritte umfasst:
(i) Modifizieren von primären CD4⁺-T-Zellen, die von mindestens drei verschiedenen T-Zell-Spendern erhalten wurden, wobei die CD4⁺-T-Zellen jedes Spenders durch Einführen eines exogenen Polynucleotids, das für IL-10 kodiert, separat modifiziert werden und
(ii) Poolen der genetisch modifizierten CD4⁺-T-Zellen,
somit Erhalten der Polydonor-CD4^{IL-10}-Zellen.

10. Verfahren nach Anspruch 9, das ferner den folgenden Schritt vor Schritt (i), nach Schritt (i), nach Schritt (i) und vor Schritt (ii), oder nach Schritt (ii) umfasst:
Inkubieren der primären CD4⁺-T-Zellen in Gegenwart von:
(a) einem Anti-CD3-Antikörper und Anti-CD28-Antikörper; oder
(b) mit Anti-CD3-Antikörper und Anti-CD28-Antikörper beschichteten Kügelchen,
wobei das Verfahren optional das Inkubieren der primären CD4⁺-T-Zellen in Gegenwart von IL-2 umfasst.

11. Verfahren nach Anspruch 9 oder Anspruch 10, wobei das exogene Polynucleotid ferner ein Segment umfasst, das für einen Selektionsmarker kodiert, wobei der kodierte Selektionsmarker optional ΔNGFR ist;
wobei das Verfahren ferner den folgenden Schritt, nach Schritt (i), umfasst:
Isolieren der genetisch modifizierten CD4⁺-T-Zellen, die den Selektionsmarker exprimieren, somit Erzeugen einer angereicherten Population von genetisch modifizierten CD4⁺-T-Zellen.

12. Verfahren nach einem der Ansprüche 9-11, wobei in Schritt (i) die primären CD4⁺-T-Zellen aus einem oder mehreren gefrorenen Lagerbeständen erhalten werden.

13. Population von CD4⁺-Zellen nach einem der Ansprüche 1-8 zur Verwendung in einem Verfahren zur Behandlung eines Patienten, bei dem eine Immuntoleranz aufgebaut werden soll, wobei das Verfahren den folgenden Schritt umfasst:
Verabreichen der Polydonor-CD4^{IL-10}-Zellen an den Patienten.

14. Population von CD4⁺-Zellen zur Verwendung nach Anspruch 13, wobei das Verfahren ferner den folgenden Schritt umfasst:
Verabreichen hämatopoetischer Stammzellen (HSC) eines HSC-Spenders an den Patienten entweder vor oder nach der Verabreichung der gepoolten CD4^{IL-10}-Zellen.

15. Population von CD4⁺-Zellen zur Verwendung nach Anspruch 13, wobei der Patient Folgendes aufweist:
(a) eine entzündliche oder Autoimmunkrankheit, wobei die entzündliche oder Autoimmunkrankheit optional aus der Gruppe ausgewählt ist, die aus Folgenden besteht: Typ-1-Diabetes, Morbus Crohn, Autoimmunuveitis, rheumatoider Arthritis, Psoriasis, Psoriasisarthritis, multipler Sklerose, systemischem Lupus, Reizdarmsyndrom, Morbus Addison, Graves-Krankheit, Sjögren-Syndrom, Hashimoto-Thyreoiditis, Myasthenia gravis, Autoimmunvaskulitis, perniziöser Anämie, Colitis ulcerosa, bullösen Erkrankungen, Sklerodermie und Zöliakie;
(b) eine Erkrankung oder Störung unter Beteiligung von NLPR3-Inflammasom-Hyperaktivität;
(c) Typ-2-Diabetes, eine neurodegenerative Erkrankung, eine Herz-Kreislauf-Erkrankung oder Reizdarmsyndrom; und/oder
(d) eine allergische oder atopische Erkrankung, wobei die allergische oder atopische Erkrankung optional aus der Gruppe ausgewählt ist, die aus Folgenden besteht: Asthma, atopischer Dermatitis und Rhinitis.

16. Population von CD4⁺-Zellen zur Verwendung nach Anspruch 13, wobei das Verfahren ferner den Schritt der Organtransplantation des Patienten umfasst, entweder vor oder nach der Verabreichung der Population von CD4⁺-T-Zellen.

17. Population von CD4⁺-Zellen nach einem der Ansprüche 1-8 zur Verwendung in einem Verfahren zur Behandlung eines Blutkrebses, wobei das Verfahren Folgendes umfasst:
Verabreichen einer Menge von Polydonor-CD4^{IL-10}-Zellen an einen Blutkrebspatienten, die ausreicht, um eine Antikrebswirkung zu induzieren,
wobei die Polydonor-CD4^{IL-10}-Zellen durch vektorvermittelten Gentransfer der kodierenden Sequenz von humanem IL-10 unter Steuerung durch einen konstitutiven Promoter genetisch modifiziert werden.

18. Population von CD4⁺-Zellen zur Verwendung nach Anspruch 17, wobei:
(a) die Polydonor-CD4^{IL-10}-Zellen dem Patienten vor oder nach der Verabreichung einer allogenen hämatopoetischen Stammzellentransplantation (Allo-HSCT) verabreicht werden und wobei die Menge der dem Patienten verabreichten Polydonor-CD4^{IL-10}-Zellen ausreicht, um eine GvHR zu supprimieren, ohne die Graft-versus-Leukämie(GvL)- oder Graft-versus-Tumor(GvT)-Wirksamkeit der Allo-HSCT zu supprimieren; und/oder
(b) der Blutkrebs eine myeloische Leukämie ist.

19. Population von CD4⁺-Zellen zur Verwendung nach Anspruch 13, wobei der Patient an Morbus Crohn leidet.

## Revendications

1. Population de lymphocytes T CD4⁺ qui ont été génétiquement modifiés pour comprendre un polynucléotide exogène codant l'IL-10, dans laquelle :
(a) les lymphocytes T CD4⁺ ont été obtenus à partir d'au moins trois différents donneurs de lymphocytes T (lymphocytes CD4^{IL-10} issus de plusieurs donneurs) ; et
(b) le polynucléotide exogène est intégré dans le génome nucléaire des lymphocytes T.

2. Population de lymphocytes T CD4⁺ selon la revendication 1, les lymphocytes T CD4⁺ de la population ayant collectivement six, sept, huit, neuf, dix, onze, douze, ou plus différents haplotypes d'antigènes leucocytaires humains (HLA).

3. Population de lymphocytes T CD4⁺ selon la revendication 1, dans laquelle :
(a) tous les lymphocytes T CD4⁺ de la population ont :
(i) une correspondance d'au moins 5/10, 6/10, 7/10, 8/10 ou 9/10 les uns avec les autres au niveau des locus HLA-A, HLA-B, HLA-C, HLA-DRB1 et HLA-DQB1 ;
(ii) une correspondance d'au moins 4/8, 5/8, 6/8, 7/8 ou 8/8 les uns avec les autres au niveau des locus HLA-A, HLA-B, HLA-C et HLA-DRB1 ;
(iii) une correspondance de 2/2 les uns avec les autres au niveau du locus HLA-A ;
(iv) une correspondance de 2/2 les uns avec les autres au niveau du locus HLA-B ;
(v) une correspondance de 2/2 les uns avec les autres au niveau du locus HLA-C ; et/ou
(vi) tous les lymphocytes T CD4⁺ de la population ont une correspondance d'au moins 3/4 ou 4/4 les uns avec les autres au niveau des locus HLA-DRB1 et HLA-DQB1 ; et/ou
(b) tous les lymphocytes T CD4⁺ de la population ont un allèle A*02.

4. Population de lymphocytes T CD4⁺ selon la revendication 1 ou la revendication 2, dans laquelle le polynucléotide exogène comprend en outre :
(a) une séquence codant un marqueur de sélection, le marqueur de sélection étant optionnellement ΔNGFR ; et/ou
(b) des séquences de vecteurs lentiviraux.

5. Population de lymphocytes T CD4⁺ selon l'une quelconque des revendications ci-dessus, dans laquelle :
(a) au moins 90 % des lymphocytes T CD4⁺ de la population expriment l'IL-10 ;
(b) les lymphocytes T CD4⁺ génétiquement modifiés expriment constitutivement au moins 100 pg, 200 pg, 500 pg, 1 ng, 5 ng, 10 ng ou 50 ng d'IL-10 par 10⁶ des lymphocytes T CD4⁺/ml de milieu de culture ; et/ou
(c) les lymphocytes T CD4⁺ génétiquement modifiés expriment au moins 1 ng, 2 ng, 5 ng, 10 ng, 100 ng, 200 ng ou 500 ng d'IL-10 par 10⁶ des lymphocytes T CD4⁺/ml après activation avec des anticorps anti-CD3 et anti-CD28.

6. Population de lymphocytes T CD4⁺ selon la revendication 4(a), dans laquelle au moins 90 %, au moins 95 % ou au moins 98 % des lymphocytes T CD4⁺ de la population expriment le marqueur de sélection provenant du polynucléotide exogène.

7. Population de lymphocytes T CD4⁺ selon l'une quelconque des revendications ci-dessus, dans laquelle les lymphocytes T CD4⁺ génétiquement modifiés expriment CD49b, LAG-3, TGF-β, IFNγ, GzB, la perforine, CD18, CD2, CD226 et/ou l'IL-22.

8. Population de lymphocytes T CD4⁺ selon l'une quelconque des revendications ci-dessus, dans laquelle les lymphocytes T CD4⁺ sont présents dans :
(a) une suspension congelée ; ou
(b) une suspension liquide, la suspension liquide ayant optionnellement été congelée au préalable.

9. Procédé de production de lymphocytes CD4^{IL-10} issus de plusieurs donneurs, comprenant les étapes qui consistent à :
(i) modifier des lymphocytes T CD4⁺ primaires qui ont été obtenus à partir d'au moins trois différents donneurs de lymphocytes T, les lymphocytes T CD4⁺ de chaque donneur étant modifiés séparément par l'introduction d'un polynucléotide exogène codant l'IL-10, et
(ii) regrouper les lymphocytes T CD4⁺ génétiquement modifiés,
en obtenant ainsi les lymphocytes CD4^{IL-10} issus de plusieurs donneurs.

10. Procédé selon la revendication 9, comprenant en outre, avant l'étape (i), après l'étape (i), après l'étape (i) et avant l'étape (ii), ou après l'étape (ii), l'étape qui consiste à :
mettre en incubation les lymphocytes T CD4⁺ primaires en présence de :
(a) un anticorps anti-CD3, et un anticorps anti-CD28 ; ou
(b) de billes enrobées d'anticorps anti-CD3 et d'anticorps anti-CD28,
le procédé comprenant optionnellement en outre la mise en incubation des lymphocytes T CD4⁺ primaires en présence d'IL-2.

11. Procédé selon la revendication 9 ou la revendication 10, dans lequel le polynucléotide exogène comprend en outre un segment codant un marqueur de sélection, optionnellement dans lequel le marqueur de sélection codé est ΔNGFR ;
le procédé comprenant optionnellement en outre, après l'étape (i), l'étape qui consiste à :
isoler les lymphocytes T CD4⁺ génétiquement modifiés exprimant le marqueur de sélection, en produisant ainsi une population enrichie de lymphocytes T CD4⁺ génétiquement modifiés.

12. Procédé selon l'une quelconque des revendications 9 à 11, dans lequel, à l'étape (i), les lymphocytes T CD4⁺ primaires sont obtenus à partir d'un ou de plusieurs stocks congelés.

13. Population de lymphocytes CD4⁺ selon l'une quelconque des revendications 1 à 8, destinée à une utilisation dans une méthode de traitement d'un patient nécessitant une tolérisation immunitaire, la méthode comprend l'étape qui consiste à :
administrer les lymphocytes CD4^{IL-10} issus de plusieurs donneurs au patient.

14. Population de lymphocytes CD4⁺ destinée à une utilisation selon la revendication 13, la méthode comprenant en outre l'étape qui consiste à :
administrer des cellules souches hématopoïétiques (CSH) d'un donneur de CSH au patient soit avant, soit après l'administration des lymphocytes CD4^{IL-10} provenant de donneurs qui ont été regroupés.

15. Population de lymphocytes CD4⁺ destinée à une utilisation selon la revendication 13, le patient présentant :
(a) une maladie inflammatoire ou auto-immune, la maladie inflammatoire ou autoimmune étant optionnellement sélectionnée dans le groupe constitué d'un diabète de type 1, d'une maladie de Crohn, d'une uvéite auto-immune, d'une polyarthrite rhumatoïde, d'un psoriasis, d'une arthrite psoriasique, d'une sclérose en plaques, d'un lupus disséminé, d'une maladie inflammatoire de l'intestin, d'une maladie d'Addison, d'une maladie de Graves, d'un syndrome de Sjögren, d'une thyroïdite de Hashimoto, d'une myasthénie grave, d'une vascularite auto-immune, d'une anémie pernicieuse, d'une colite ulcéreuse, de maladies bulleuses, d'une sclérodermie et d'une maladie cœliaque ;
(b) une maladie ou une affection impliquant une hyperactivité de l'inflammasome NLPR3 ;
(c) un diabète de type 2, une maladie neurodégénérative, une maladie cardiovasculaire ou une maladie inflammatoire de l'intestin ; et/ou
(d) une maladie allergique ou atopique, la maladie allergique ou atopique étant optionnellement sélectionnée dans le groupe constitué d'un asthme, d'une dermatite atopique et d'une rhinite.

16. Population de lymphocytes CD4⁺ destinée à une utilisation selon la revendication 13, la méthode comprenant en outre l'étape qui consiste en une transplantation d'organe au patient, avant ou après l'administration de la population de lymphocytes T CD4⁺.

17. Population de lymphocytes CD4⁺ selon l'une quelconque des revendications 1 à 8, destinée à une utilisation dans une méthode de traitement d'un cancer hématologique, la méthode comprenant :
l'administration à un patient atteint d'un cancer hématologique d'une quantité de lymphocytes CD4^{IL-10} issus de plusieurs donneurs suffisante pour induire un effet anticancéreux,
les lymphocytes CD4^{IL-10} issus de plusieurs donneurs étant génétiquement modifiés par transfert de gènes médié par vecteur de la séquence codante de l'IL-10 humaine sous le contrôle d'un promoteur constitutif.

18. Population de lymphocytes CD4⁺ destinée à une utilisation selon la revendication 17, dans laquelle :
(a) les lymphocytes CD4^{IL-10} issus de plusieurs donneurs sont administrés au patient avant ou après l'administration d'une greffe allogénique de cellules souches hématopoïétiques (GCSH allo), et la quantité de lymphocytes CD4^{IL-10} issus de plusieurs donneurs administrée au patient est suffisante pour supprimer la maladie du greffon contre l'hôte (GvHD) sans supprimer l'effet du greffon contre la leucémie (GvL) ou du greffon contre la tumeur (GvT) de la GCSH allo ; et/ou
(b) le cancer hématologique est une leucémie myéloïde.

19. Population de lymphocytes CD4⁺ destinée à une utilisation selon la revendication 13, le patient étant atteint de la maladie de Crohn.
